(19) Europäisches Patentamt
European Patent Office
Office européen des brevets

(11) **EP 3 535 589 B1**

(12) **FASCICULE DE BREVET EUROPEEN**

(45) Date de publication et mention
de la délivrance du brevet:
**04.12.2024 Bulletin 2024/49**

(21) Numéro de dépôt: **17801082.3**

(22) Date de dépôt: **03.11.2017**

(51) Classification Internationale des Brevets (IPC):
**G01N 33/80** *(2006.01)* **G01N 15/14** *(2024.01)*
**G01N 33/72** *(2006.01)* **G01N 15/10** *(2024.01)*
**G01N 15/01** *(2024.01)*

(52) Classification Coopérative des Brevets (CPC):
**G01N 33/721; G01N 15/1459; G01N 33/80;**
G01N 2015/012; G01N 2015/1006; G01N 2800/52

(86) Numéro de dépôt international:
**PCT/FR2017/053015**

(87) Numéro de publication internationale:
**WO 2018/083426 (11.05.2018 Gazette 2018/19)**

(54) **PROCÉDÉ DE DETERMINATION DE LA TENEUR EN HÉMOGLOBINE F D'UNE CELLULE ÉRYTHROÏDE**

VERFAHREN ZUR BESTIMMUNG DES HEMOGLOBIN-F-INHALTS EINER ERYTHROIDZELLE

METHOD FOR DETERMINING THE HEMOGLOBIN F CONTENT OF AN ERYTHROID CELL

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **04.11.2016 FR 1660713**

(43) Date de publication de la demande:
**11.09.2019 Bulletin 2019/37**

(73) Titulaires:
• **Université Paris-Est Créteil Val de Marne
94000 Créteil (FR)**
• **Institut National de la Santé et de la
Recherche Médicale (INSERM)
75013 Paris (FR)**
• **Institut National Transfusion Sanguine
75015 Paris (FR)**
• **Etablissement Français du Sang
93218 La Plaine Saint Denis (FR)**
• **Assistance Publique - Hôpitaux de Paris
75004 Paris (FR)**

(72) Inventeurs:
• **CAMBOT, Marie
94240 L'Hay Les Roses (FR)**
• **VANDEMEULEBROUCK, Gaetana
75020 Paris (FR)**
• **NOIZAT PIRENNE, France
75012 Paris (FR)**
• **BARTOLUCCI, Pablo
94240 L'Hay Les Roses (FR)**
• **RAKOTOSON, Marie Georgine
Antananarivo (MG)**
• **GALACTEROS, Frédéric
94440 Santeny (FR)**
• **HEBERT, Nicolas
91340 Ollainville (FR)**

(74) Mandataire: **Icosa
83 avenue Denfert-Rochereau
75014 Paris (FR)**

(56) Documents cités:
**WO-A1-03/021275**

• **AMOYAL I. ET AL.: "Hemoglobin switch in the
newborn: A flow cytometry analysis",
NEONATOLOGY, vol. 91, no. 1, 2007, pages 61 -
68, XP009194167**
• **MUNDEE Y. ET AL.: "Simplified flow cytometric
method for fetal hemoglobin containing red blood
cells", CYTOMETRY, vol. 42, no. 6, 15 December
2000 (2000-12-15), pages 389 - 393, XP002571315**

- **MUNDEE Y. ET AL.: "Flow cytometric method for simultaneous assay of foetal haemoglobin containing red cells, reticulocytes and foetal haemoglobin containing reticulocytes", CLIN. LAB. HAEM., vol. 23, no. 3, June 2001 (2001-06-01), pages 149 - 154, XP002571316**
- **AMOYAL I. ET AL.: "Flow cytometric analysis of fetal hemoglobin in erythroid precursors of beta-thalassemia", CLIN. LAB. HAEM., vol. 26, no. 3, June 2004 (2004-06-01), pages 187 - 193, XP055367435**
- **RAKOTOSON M.G. ET AL.: "Biological parameters predictive of percent dense red blood cell decrease under hydroxyurea", ORPHANET J. RARE DIS., vol. 10, no. 1, 9 May 2015 (2015-05-09), pages 57, XP021221659**
- **DI LIBERTO G. ET AL.: "Dense red blood cell and oxygen desaturation in sickle-cell disease", AM. J. HEMATOL., vol. 91, no. 10, October 2016 (2016-10-01), pages 1008 - 1013, XP055366714**

**Description**

**Domaine technique**

L'invention concerne un nouveau procédé de détermination *in vitro* de la teneur en hémoglobine F (HbF) de chaque cellule érythroïde d'un ensemble de cellules érythroïdes, de préférence de chaque globule rouge d'un ensemble de globules rouges, par cytométrie de flux. L'invention concerne également un nouveau procédé de détermination *in vitro* de la quantité de globules rouges transfusés chez un patient et un procédé de suivi de l'efficacité thérapeutique d'un traitement de la drépanocytose ou de la β-thalassémie.

**Arrière-plan technologique**

La drépanocytose est une maladie génétique de l'hémoglobine qui a été historiquement caractérisée par la déformation des globules rouges (GR) en réponse à une diminution de la pression partielle en oxygène. Les globules rouges perdent leur forme biconcave (ou discocyte) et se présentent sous forme allongée, pointue ou en faucille d'où l'appellation anémie falciforme ou « sickle cell disease » en anglais.

La drépanocytose est due à une mutation d'un seul nucléotide (GAG→GTG) sur le codon 6 du gène β localisé sur le bras court du chromosome 11 (11p 15.5). Cette mutation se traduit par une modification structurale de la chaîne β de l'hémoglobine qui consiste en un remplacement de l'acide glutamique en position 6 par une valine pour former l'HbS. Le syndrome drépanocytaire majeur comprend 3 génotypes : une forme homozygote (SS), et 2 formes hétérozygotes (SB$^0$Thal et SC qui associe la mutation βS et une mutation C caractérisée par la substitution de l'acide glutamique en position 6 par une lysine).

Le phénotype clinique de la drépanocytose est caractérisé par une grande variabilité interindividuelle d'une part et d'autre part par une variabilité entre chaque évènement pour un même patient. Les principales causes d'hospitalisation sont les crises vasoocclusives (CVO) et le syndrome thoracique aigu (STA) qui peuvent se présenter à tout âge. Le STA peut toucher jusqu'à 40% des patients drépanocytaires et représente l'une des principales causes de mortalité chez l'adulte. Les globules rouges denses ou « dense red blood cells » (DRBC) en anglais et les « irreversibly sickled cells » (ISC) constituent une sous population de globules rouges SS très pathogène de par leur forte concentration en HbS qui est le principal facteur déterminant de la polymérisation. Les DRBC ont une forte tendance à se polymériser et sont des acteurs majeurs des mécanismes physiopathologiques de la drépanocytose. Par leur faible déformabilité associée à une augmentation importante de la viscosité, les DRBC contribuent principalement à l'occlusion des capillaires et des veinules post capillaires et au ralentissement du flux sanguin favorisant l'apparition des phénomènes vasoocclusifs.

En 2010, environ 5 millions de porteurs du trait drépanocytaire et 312 000 enfants drépanocytaires ont été recensés à l'échelle mondiale, avec une forte densité retrouvée dans les régions africaines. En 2011, l'OMS a estimé une prévalence des porteurs du trait drépanocytaire allant jusqu'à 5% de la population mondiale. La drépanocytose représente aujourd'hui la maladie génétique la plus répandue en France avec une fréquence allant jusqu'à une naissance sur 700 en Métropole et 1/2300 avec les départements d'Outre-Mer (Bulletin Epidémiologique Hebdomadaire 3 juillet 2012/ 12 mai 2015). Les taux de mortalité chez les enfants de moins de 5 ans ont nettement diminué depuis les dernières décennies grâce au dépistage néonatal et à l'introduction des traitements préventifs des infections qui constituent les principales causes de mortalité. En France, une amélioration de l'espérance de vie des patients drépanocytaires a été également observée durant les 20 dernières années avec un taux de mortalité réduit à 35% (Bulletin Epidémiologique Hebdomadaire 10 mars 2015). Cependant, le manque de dépistage précoce et l'accès limité aux traitements et soins de qualité continuent à faire augmenter les incidences de mortalité chez les enfants dans les pays en voie de développement.

L'observation d'une forme asymptomatique de la drépanocytose chez des patients SS a conduit à différentes études dans le but de déterminer l'effet protecteur de l'hémoglobine F (HbF). En effet, l'expression clinique de la drépanocytose est atténuée en période périnatale au cours de laquelle l'HbF représente la forme prédominante. On observe également une forme bénigne chez l'adulte présentant des traits génétiques conduisant à une surexpression de l'HbF. Ces patients présentent rarement une manifestation clinique sévère et bénéficient d'une meilleure espérance de vie. L'effet protecteur de l'HbF consiste principalement en l'inhibition de la formation du polymère de désoxy HbS. Puisque la polymérisation dépend essentiellement de la concentration corpusculaire moyenne en hémoglobine (CCMH), la réduction de la concentration intracellulaire en HbS constitue le principal facteur améliorant l'expression clinique. Les études in vitro effectuées sur des solutions d'hémoglobines ont permis de déterminer les mécanismes d'inhibition de la polymérisation par les différents variants d'hémoglobine (HbA, HbA$_2$, HbF, ...). Un effet inhibiteur plus important est obtenu avec l'HbF. A cet effet antipolymérisant s'ajoute une forte affinité de l'HbF à l'oxygène qui fournit une meilleure protection des globules rouges. Chez les patients drépanocytaires, l'HbF est exprimée de manière hétérogène, l'effet protecteur de l'HbF procure aux globules rouges contenant un taux élevé en HbF une durée de vie plus longue, jusqu'à environ 90 jours, tandis que les globules rouges SS qui contiennent peu d'HbF sont rapidement éliminés de la circulation générale.

A ce jour, l'augmentation de l'HbF constitue donc l'un des principaux objectifs thérapeutiques dans la prise en charge de la drépanocytose.

Néanmoins, l'augmentation de la teneur moyenne en HbF n'est pas toujours associée à une amélioration clinique chez les patients drépanocytaires. A l'inverse, une amélioration clinique peut être observée à de faibles teneurs moyennes en HbF. Ces observations suggèrent l'existence d'une teneur seuil en HbF dans chaque globule rouge permettant d'inhiber la polymérisation de l'HbS et évoquent la nécessité d'obtenir un dépassement de ce seuil avec comme résultante une distribution plus homogène de l'HbF dans les globules rouges, afin que chaque globule rouge bénéficie de l'effet protecteur de l'HbF. Il a par ailleurs été montré qu'en l'absence de traitement, la distribution de l'HbF dans les globules rouges SS est hétérogène. La détermination de la teneur en HbF de chaque globule rouge revêt donc une importance capitale.

Depuis quelques décennies plusieurs techniques ont été développées afin de déterminer la teneur en HbF de chaque globule rouge. Ces techniques étaient basées initialement sur des méthodes chimiques (Betke K, Marti HR, Schlicht I. Estimation of small percentages of foetal haemoglobin. Nature. 1959;184(Suppl 24):1877-8), puis sur des méthodes immunologiques (Navenot JM, Merghoub T, Ducrocq R, Muller JY, Krishnamoorthy R, Blanchard D. New method for quantitative détermination of fetal hemoglobin-containing red blood cells by flow cytometry: application to sickle-cell disease. Cytometry. 1998;32(3):186-90 ; Mundee Y. et al., Simplified flow cytométrie method for fetal hemoglobin containing red blood cells, Cytometry, 2000, 42(6):389-393), avaient pour but de détecter une population particulière de globules rouges contenant un taux élevé en HbF appelés « cellules F ». Bien que très utilisées dans le diagnostic des incompatibilités foeto-maternelles et dans la compréhension des hémoglobinopathies, ces méthodes sont peu sensibles et se limitent à une mesure qualitative des cellules F (globules rouges positifs en microscopie par immunofluorescence, immunodiffusion ou en cytométrie de flux, quel que soit le niveau d'expression de l'HbF). Jusque-là, l'étude de la teneur en HbF dans chaque globule rouge était basée sur cette définition approximative des cellules F.

Il existe donc un réel besoin de mettre au point une méthode facile à mettre en oeuvre pour déterminer précisément la teneur en HbF dans chaque globule rouge d'un ensemble de globule rouge afin de pouvoir expliquer les variabilités de réponse clinique, anticiper ces variabilité de réponse clinique et ainsi adapter le traitement. Cela permettrait également de développer de nouvelles approches thérapeutiques de la drépanocytose ou de la β-thalassémie visant à augmenter la teneur en HbF dans chaque globule rouge et pas seulement la teneur moyenne en HbF chez un patient.

## Résumé de l'invention

La présente invention vise à proposer un nouveau procédé de détermination de la teneur en hémoglobine F (HbF) de chaque globule rouge d'un ensemble de globules rouges, utile dans le diagnostic et le suivi thérapeutique de la drépanocytose ou de la β-thalassémie.

Selon un premier aspect, l'invention concerne un procédé de détermination *in vitro* de la teneur en hémoglobine F (HbF) de chaque globule rouge d'un ensemble de globules rouges comprenant les étapes de :

   a) isoler des globules rouges à partir d'un échantillon ;
   b) perméabiliser la membrane des globules rouges isolés;
   c) marquer l'HbF des globules rouges obtenus à l'étape b) avec un anticorps anti-HbF conjugué à un fluorochrome ;
   d) mesurer par cytométrie de flux l'intensité de fluorescence (MFI) de chaque globule rouge d'un ensemble de globules rouges
   e) déterminer la teneur en hémoglobine F de chaque globule rouge de l'ensemble de globules rouges.

Selon un deuxième aspect, l'invention concerne un procédé de détermination *in vitro* de la quantité de globules rouges transfusés chez un patient, comprenant :

   a) l'obtention d'un échantillon contenant des globules rouges à partir d'un patient ;
   b) la détermination de la teneur en hémoglobine F (HbF) de chaque globule rouge d'un ensemble de globules rouges dudit échantillon selon le procédé de l'invention ;
   c) l'utilisation des résultats de l'étape b) pour déterminer la quantité de globules rouges transfusés chez un patient, lesdits globules rouges transfusés ayant une teneur en HbF substantiellement égale à zéro (= 0 pg).

Selon un troisième aspect, l'invention concerne un procédé *in vitro* de suivi de l'efficacité thérapeutique d'un traitement de la drépanocytose ou de la β-thalassémie comprenant :

   a) l'obtention d'un échantillon contenant des globules rouges à partir d'un patient ayant subi un traitement de la drépanocytose ;

b) la détermination de la teneur en hémoglobine F (HbF) de chaque globule rouge d'un ensemble de globules rouges dudit échantillon selon le procédé de l'invention appliqué aux globules rouges ;

c) l'utilisation des résultats de l'étape b) dans la détermination de l'efficacité thérapeutique du traitement de la drépanocytose ou de la β-thalassémie, dans lequel une efficacité thérapeutique est observée lorsqu'au moins un pourcentage prédéterminé des globules rouges de l'ensemble de globules rouges ont une teneur en HbF qui dépasse un seuil de référence et/ou lorsqu'on observe une diminution de la quantité de globules rouges ayant une teneur en HbF <2pg.

## Description détaillée de l'invention

Définitions

Les hémoglobines humaines sont composées de 4 sous unités de chaînes polypeptidiques identiques deux à deux. Les sous unités varient selon les types d'hémoglobine et on peut distinguer 4 hémoglobines normales chez l'homme :

- les hémoglobines embryonnaires : hémoglobine Gower I ($\zeta_2\varepsilon_2$}, hémoglobine Portland {$\zeta_2\gamma_2$) et hémoglobine Gower II ($\alpha_2\varepsilon_2$) ;
- l'HbF ($\alpha_2\gamma_2$);
- l'hémoglobine adulte (HbA) ($\alpha_2\beta_2$);
- l'hémoglobine A2 (HbA2) ($\alpha_2\delta_2$).

Le terme « HbF » désigne donc une protéine composée de deux sous unités de chaînes $\alpha$ (alpha) et deux sous unités de chaîne chaînes $\gamma$ (gamma). L'HbF est composée de deux types de chaînes $\gamma$ : G$\gamma$ et A$\gamma$ qui se distinguent par leur résidu en position 136 correspondant à une Glycine pour la chaîne G$\gamma$ et une Alanine pour A$\gamma$. Il existe des formes mineures d'HbF dont l'HbF1 qui représente 10% d'hémoglobine totale chez le foetus. Elle contient une sous unité $\gamma^x$ dont l'extrémité N terminale est substituée par un groupement acétyl. D'autres formes d'HbF peuvent être observées mais à très faible quantité.

Au sens de l'invention, le terme « teneur » doit être pris en son sens premier, c'est-à-dire la quantité de l'entité d'intérêt. La teneur peut être exprimée en teneur massique, par exemple en picogramme (pg).

Au sens de l'invention, les termes « teneur en HbF de chaque globule rouge » désignent la quantité d'HbF dans chaque globule rouge, pris individuellement. De préférence la teneur (ou quantité) est exprimée en masse, avantageusement en picogramme (pg). Par exemple, la détermination de la teneur en HbF de chaque globule rouge d'un ensemble de 10 globules rouges corespond à l'obtention de 10 valeurs de quantité d'HbF, correspondant à la teneur en HbF de chacun des 10 globules rouges pris individuellement.

Les termes « cellule érythroïde » ou « cellules érythroïdes » désignent des cellules qui expriment de l'hémoglobine pour lesquelles il est possible de mesurer la présence d'hémoglobine foetale (HbF) parmi un ensemble desdites cellules. Les « cellules érythroïdes »désignent, les cellules érythroïdes qui se différencient au stade de l'érythropoïèse tardive, par exemple les érythroblastes, les réticulocytes ou les globules rouges. Lesdites cellules de l'érythropoïèse tardive sont des cellules pouvant être non circulantes, c'est-à-dire issues de la moelle osseuse (par exemple les érythroblastes) ou des cellules circulantes, c'est-à-dire des cellules sanguines (par exemple les réticulocytes ou les globules rouges) ou des cellules dérivées de cellules érythroïdes, par exemple des cellules érythroïdes recombinantes. Les cellules érythroïdes sont susceptibles de contenir de l'HbF. Les termes « cellule érythroïde » et « cellules érythroïdes » peuvent être respectivement substitués, par exemple, par « globule rouge » ou « globules rouges ». L'invention concerne un procédé de détermination *in vitro* de la teneur en hémoglobine F (HbF) de chaque globule rouge d'un ensemble de globules rouges comprenant les étapes de :

a) isoler des globules rouges à partir d'un échantillon ;

b) perméabiliser la membrane des globules rouges isolés ;

c) marquer l'HbF des globules rouges obtenus à l'étape b) avec un anticorps anti-HbF conjugué à un fluorochrome ;

d) mesurer par cytométrie de flux l'intensité de fluorescence (MFI) de chaque globule rouge d'un ensemble de globules rouges ;

e) déterminer la teneur en hémoglobine F de chaque globule rouge de l'ensemble de globules rouges.

Les termes « ensemble de cellules érythroïdes », correspond à un ensemble de cellules érythroïdes contenues dans un échantillon de cellules érythroïdes. L'ensemble de cellules érythroïdes pouvant aller jusqu'à 100% des cellules érythroïdes présentes dans l'échantillon, c'est-à-dire la totalité des cellules érythroïdes présentes dans l'échantillon.

Ainsi, l'ensemble de cellules érythroïdes correspond à entre 100% et 0%, 0% étant exclu, des cellules érythroïdes présentes dans l'échantillon.

Particulièrement, l'ensemble de cellules érythroïdes comprends au moins 10 000 cellules érythroïdes, de préférence au moins 100 000 cellules érythroïdes, de préférence entre 50 000 et 100 000 cellules érythroïdes. La teneur en hémoglobine F peut être déterminée pour chacune des cellules érythroïdes d'un ensemble d'au moins 10 000 cellules érythroïdes, de préférence d'au moins 100 000 cellules érythroïdes, de préférence entre 50 000 et 100 000 cellules érythroïdes.

Au sens de l'invention, les termes « cytométrie de flux » désignent une technique bien connue de l'homme du métier permettant de faire défiler des particules, molécules ou cellules à grande vitesse dans le faisceau d'un laser, en les comptant et en les caractérisant. C'est la lumière réémise par les particules, molécules ou cellules (par diffusion ou fluorescence) qui permet de les caractériser suivant le ou les critères souhaités. Généralement les particules, molécules ou cellules sont marquées avec un fluorochrome qui absorbe l'énergie du laser et qui réémet l'énergie absorbée sous forme de photons d'une longueur d'onde plus élevée. Dans le cadre de l'invention, la lumière réémise est obtenue par un marquage spécifique de l'HbF présente dans les cellules érythroïdes avec un anticorps anti-HbF conjugué à un fluorochrome.

Au sens de l'invention, le terme « fluorochrome » (ou « fluorophore ») désigne une substance chimique capable d'émettre de la lumière de fluorescence après excitation par un laser. Dans le cadre de l'invention, le fluorochrome est couplé à un anticorps anti-HbF. L'homme du métier dispose d'un large choix de fluorochromes adaptés à la cytométrie de flux. Tous les fluorochromes qui peuvent être couplés à un anticorps peuvent être utilisés dans le cadre de la présente invention. Dans un mode de réalisation particulier, le fluorochrome est la fluorescéine isothiocyanate (FITC), la phycoérythrine (PE) ou un de leurs dérivés, de préférence la PE. La PE, par ses propriétés conformationnelles (encombrement stérique) présente l'avantage d'avoir un ratio PE : anticorps proche de 1. Les chimies de couplage des anticorps sont bien connues de l'homme du métier et la présente invention n'est pas limitée à une chimie de couplage particulière.

Le terme « anticorps » tel qu'il est utilisé ici fait référence à des molécules d'immunoglobulines ou d'autres molécules qui comprennent au moins un domaine de liaison à un antigène. Il englobe notamment des anticorps entiers, des fragments d'anticorps comprenant un domaine de liaison à l'antigène (e.g. Fab, Fab' et F(ab)2, scFv, les fragments comprenant soit un domaine VL soit un domaine VH), des anticorps monoclonaux, des anticorps polyclonaux, des anticorps chimériques, des anticorps humanisés, des anticorps primatisés, des anticorps monospécifiques, des anticorps multispécifiques, des anticorps mono-chaine (e.g. de type camélidé). Les anticorps selon l'invention peuvent être des anticorps de tout type, par exemple, IgG, IgE, IgM, IgD, IgA et IgY, de toute classe, par exemple, IgG1, IgG2, IgG3, IgG4, IgA1 et IgA2 ou de toute sous-classe. Dans le cadre de la présente invention, l'anticorps est un anticorps anti-HbF, de préférence un anticorps anti-HbF dirigé contre la chaîne gamma de l'HbF. Dans un mode de réalisation particulier, l'anticorps est un anticorps monoclonal.

Généralement, les anticorps monoclonaux peuvent être obtenus par la technique des hybridomes dont le principe général est rappelé ci-après. Dans un premier temps, on immunise un animal, généralement une souris avec l'antigène d'intérêt (par exemple l'HbF humaine) ou un fragment de l'antigène d'intérêt (par exemple la chaîne gamma de l'HbF humaine), dont les lymphocytes B sont alors capables de produire des anticorps contre ledit antigène. Ces lymphocytes producteurs d'anticorps sont ensuite fusionnés avec des cellules myélomateuses "immortelles" (par exemple murines) pour donner lieu à des hybridomes. Chaque hybridome est multiplié sous la forme de clone, chacun conduisant à la production d'un anticorps monoclonal dont les propriétés de reconnaissance vis-à-vis dudit antigène d'intérêt pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie. Les anticorps monoclonaux peuvent être également des anticorps recombinants obtenus par génie génétique, par des techniques bien connues de l'homme du métier.

Au sens de l'invention, le terme « diagnostic » désigne la détermination d'une probabilité ou d'une possibilité d'avoir une drépanocytose chez un patient.

Au sens de l'invention, le terme « échantillon » désigne un échantillon contenant des globules rouges obtenu à partir d'un patient et à partir duquel il est possible de mettre en oeuvre l'invention. Avantageusement, l'échantillon est un échantillon sanguin, de préférence un échantillon sanguin humain. L'échantillon peut être prélevé chez un patient à tout moment, par exemple par une prise de sang.

Au sens de l'invention, le terme « patient homogène » désigne un patient présentant une teneur en HbF homogène sur l'ensemble de ses globules rouges.

La teneur en HbF sur l'ensemble des globules rouges suit donc une distribution logarithmique normale. Un patient homogène peut être un patient présentant une persistance héréditaire de l'HbF, une β-thalassémie ou une β-δ-thalassémie. Ainsi le terme « patients homogènes » désigne un ensemble de patients présentant chacun une teneur en HbF homogène sur l'ensemble de leurs globules rouges, la teneur en HbF pouvant être différente d'un patient à l'autre.

Au sens de l'invention, le terme « échantillon homogène » désigne un échantillon de globules rouges obtenu à partir d'un patient homogène. Ainsi, les teneurs en HbF des globules rouges d'un échantillon homogène présentent une variance faible. C'est-à-dire que les teneurs en HbF de chacune des globules rouges de l'échantillon homogène sont concentrées autour de la teneur en HbF de l'échantillon homogène. Avantageusement, une variance faible est une variance inférieure à 15%, avantageusement inférieure à 10%, inférieure à 9%, 8%, 7%, 6%, 5%, 4%, 3%, 2%, 1%.

Procédé de détermination de la teneur en HbF d'une cellule érythroïde

Les inventeurs ont mis en évidence que la cytométrie de flux était une technologie particulièrement adaptée pour déterminer facilement et précisément la teneur en HbF de chaque globule rouge d'un ensemble de globules rouges.

Ainsi, l'invention concerne un procédé de détermination *in vitro* de la teneur en hémoglobine F (HbF) de chaque globule rouge d'un ensemble de globules rouges comprenant les étapes de :

a) isoler des globules rouges à partir d'un échantillon ;

b) perméabiliser la membrane des globules rouges isolés ;

c) marquer l'HbF des globules rouges obtenus à l'étape b) avec un anticorps anti-HbF conjugué à un fluorochrome ;

d) mesurer par cytométrie de flux l'intensité de fluorescence (MFI) de chaque globule rouge d'un ensemble de globules rouges

e) déterminer la teneur en hémoglobine F de chaque globule rouge d'un ensemble de globules rouges.

L'étape a) consiste à isoler des globules rouges à partir d'un échantillon. Cette étape consiste à purifier (ou augmenter le niveau de pureté) des globules rouges à partir d'un échantillon comprenant des globules rouges.

Cette étape permet notamment d'éliminer certains éléments (e.g. les plaquettes et les globules blancs) susceptibles d'être présents dans l'échantillon. Avantageusement, l'échantillon est un échantillon sanguin, de préférence un échantillon sanguin humain. De nombreuses techniques sont à la disposition de l'homme du métier pour isoler des globules rouges à partir d'un échantillon contenant des globules rouges.

Ces techniques sont généralement simples à mettre en oeuvre et ne présentent généralement aucune difficulté particulière pour l'homme du métier qui saura les adapter pour leur mise en oeuvre dans la présente invention. On peut par exemple citer la centrifugation, les techniques de chromatographie, le fractionnement par gradient de densité. Par exemple, (i) un échantillon de sang est centrifugé, (ii) le culot (contenant les globules rouges) est collecté et (iii) un tampon isotonique approprié (par exemple un tampon phosphate) est ajouté au culot pour obtenir une suspension de globules rouges, les étapes (i) à (iii) pouvant être répétées une ou plusieurs fois afin d'obtenir les globules rouges isolés. L'étape b) consiste à perméabiliser la membrane des globules rouges isolés.

Cette étape consiste à rendre la membrane des globules rouges suffisamment perméable pour que l'anticorps anti-HbF puisse pénétrer dans les globules rouges, tout en préservant l'intégrité des globules rouges (sans lyse des globules rouges). De nombreuses techniques sont à la disposition de l'homme du métier pour perméabiliser la membrane des globules rouges.

Ces techniques sont généralement simples à mettre en oeuvre et ne présentent généralement aucune difficulté particulière pour l'homme du métier qui saura les adapter pour leur mise en oeuvre dans la présente invention. On peut par exemple citer l'utilisation d'un agent chimique tels qu'un détergent et/ou un tensioactif (e.g. saponine, SDS et/ou Triton). Dans un mode de réalisation particulier, la membrane des globules rouges isolés est perméabilisée avec du dodécyl sulfate de sodium (SDS). Le SDS, également connu sous la dénomination laurylsulfate de sodium (LSS) ou sodium lauryl sulfate (SLS), est un détergent et tensioactif ionique fort, couramment utilisé. Généralement, le dodecyl sulfate de sodium est simplement rajouté à la suspension de globules rouges.

Dans un mode de réalisation particulier, la membrane des globules rouges isolés est fixée avant l'étape de perméabilisation. La fixation de la membrane des globules rouges permet d'éviter (ou de limiter) la lyse des globules rouges lors de l'étape de perméabilisation. Plusieurs composés chimiques sont à la disposition de l'homme du métier pour fixer la membrane des globules rouges.

Dans un mode de réalisation particulier, la membrane des globules rouges isolés est fixée avec de l'azoture de sodium et/ou du formaldéhyde. Généralement, l'azoture de sodium et/ou du formaldéhyde sont simplement rajouté sur un culot de globules rouges ou dans une suspension de globules rouges.

L'étape c) consiste à marquer l'HbF des globules rouges obtenus à l'étape b) (c'est-à-dire les globules rouges isolés dont la membrane est perméabilisée) avec un anticorps anti-HbF conjugué à un fluorochrome (également dénommé ci-après « anticorps anti-HbF »). Avantageusement, l'étape c) est réalisée dans des conditions permettant une liaison intracellulaire entre l'anticorps anti-HbF et l'HbF. Cette étape permet d'obtenir des « globules rouges marqués ». De préférence, on utilise une quantité suffisante d'anticorps anti-HbF pour pouvoir marquer l'ensemble des HbF, par exemple on utilise un excès d'anticorps anti-HbF. En particulier, L'étape c) consiste à marquer l'HbF des globules rouges obtenus à l'étape b) avec un anticorps anti-HbF conjugué à un fluorochrome dans des conditions permettant une liaison intracellulaire entre l'anticorps anti-HbF et l'HbF. C'est-à-dire que la liaison entre l'anticorps anti-HbF et l'HbF a lieu à l'intérieur du globule rouge.

Généralement, le marquage consiste à incuber les globules rouges dont la membrane a été perméabilisée avec l'anticorps anti-HbF pendant une durée suffisante pour permettre une liaison intracellulaire entre l'anticorps anti-HbF et l'HbF. La durée est généralement de quelques minutes, par exemple entre 5 min et 20 min.

Dans un mode de réalisation particulier, les globules rouges sont lavés après l'étape de marquage. Le lavage permet d'éliminer les anticorps anti-HbF qui ne seraient pas liés à l'HbF. Le lavage peut s'effectuer avec un tampon approprié, par exemple un tampon PBS. Le lavage permet également d'éliminer l'agent chimique qui a été utilisé pour perméabiliser la membrane des globules rouges. Les globules rouges ainsi lavés sont ensuite utilisés à l'étape de mesure par cytométrie de flux.

L'étape d) consiste à mesurer par cytométrie de flux l'intensité de fluorescence (MFI) de chaque globule rouge d'un ensemble de globules rouges.

Avantageusement, l'intensité de fluorescence (MFI) de chaque globule rouge d'un ensemble de globules rouges est mesurée indépendamment de l'intensité de fluorescence (MFI) des autres globule rouge de l'ensemble de globules rouges. A cette étape, un ensemble de globules rouges marqués c'est-à-dire tout ou partie des globules rouges marqués est donc analysé par cytométrie de flux. Ainsi, l'intensité de fluorescence (MFI) de chaque globule rouge de l'ensemble des globules rouges est mesurée indépendamment de l'intensité de fluorescence (MFI) des autres globules rouges de l'ensemble de globules rouges. L'intensité de fluorescence de chacun des globules rouges pris indépendamment est donc mesurée à cette étape. Dans un mode de réalisation particulier, l'intensité de fluorescence (MFI) est mesurée pour chaque globule rouge d'un ensemble d'au moins 10 000 globules rouges, de préférence d'au moins 100 000 globules rouges de préférence entre 50 000 et 100 000 globules rouges.

L'étape e) consiste à déterminer la teneur en hémoglobine F de chaque globule rouge de l'ensemble de globules rouges. La teneur en HbF est déterminée à partir de l'intensité de fluorescence mesuré par cytométrie de flux.

Ainsi, la teneur en HbF de chaque globule rouge de l'ensemble de globules rouges est déterminée indépendamment de la teneur en HbF des autres globules rouges de l'ensemble de globules rouges. La détermination de la teneur en HbF de chaque globule rouge de l'ensemble de globules rouges se fait par la comparaison de l'intensité de fluorescence de chaque globule rouge avec une courbe standard. La courbe standard permet d'associer l'intensité lumineuse mesuré pour un globule rouge à une teneur en HbF.

Par exemple, la courbe standard peut être obtenue de la manière suivante :

- on établit une droite de calibration qui permet de corréler une intensité de fluorescence à un nombre de fluorophore. Par exemple, on peut utiliser des billes conjuguées avec une quantité connue de fluorophore (billes calibrées). Cette droite de calibration permet de déterminer la quantité de fluorophore par globule rouge (ci-après fluorophore/ globule rouge).

Ainsi, en reportant la mesure de l'intensité de fluorescence de chaque globule rouge sur la droite de calibration, on peut alors déduire la quantité de fluorophore de chaque globule rouge.

On peut également utiliser le ratio fluorophore : anticorps (qui peut être 1 : 1), pour déduire le nombre d'antigène de chaque globule rouge, c'est-à-dire le nombre d'HbF de chaque globule rouge; - on détermine la teneur moyenne en HbF par globule rouge (TCMHbF) d'un échantillon sanguin obtenu à partir de patients présentant une teneur en HbF parfaitement homogène sur l'ensemble de leurs globules rouges (« patients homogènes »). La teneur en HbF sur l'ensemble de ces globules rouges suit plus précisément une distribution logarithmique normale. Ces échantillons sont appelés ci-après « échantillons homogènes ». Les patients homogènes sont par exemple des patients présentant une persistance héréditaire de l'HbF ou une β-thalassémie ou une β-δ-thalassémie. Par exemple, on peut :

o déterminer le pourcentage moyen en HbF (c'est à dire la part HbF par rapport à l'ensemble de toutes les hémoglobines, exprimée en %) de plusieurs échantillons homogènes présentant une teneur en HbF par globule rouge parfaitement différente d'un échantillon à l'autre, par exemple par chromatographie liquide haute performance (HPLC). Ce pourcentage moyen en HbF peut par exemple se calculer à partir de chromatogrammes HPLC en appliquant la formule : %HbF= (ASC HbF/ ASC total Hb)*100 où %HbF représente le pourcentage moyen en HbF sur l'ensemble des globules rouges, ASC HbF représente l'aire sous la courbe de l'HbF sur le chromatogramme, ASC total représente la somme des aires sous les courbes de chaque type d'hémoglobine ;

o déterminer la teneur moyenne en hémoglobine (c'est-à-dire toutes les hémoglobines) par globule rouge (TCMH) des échantillons homogènes.

Pour cela on divise la concentration d'hémoglobine exprimée par litre de sang (par exemple obtenue par avec un hémogramme sanguin) par le nombre de globules rouges par litre de sang (par exemple obtenu par numération des globules rouges);

    o déterminer la teneur moyenne en HbF par globule rouge d'un échantillon homogène selon la formule suivante : TCMHbF = (%HbF * TCMH)/100 ;

- on établit la courbe standard en associant TCMHbF et une mesure de l'intensité de fluorescence calibrée en fluorophore/globule rouge.

Avantageusement, la comparaison de l'intensité de fluorescence de chaque globule rouge, normalisée par rapport à la quantité de fluorophores, avec une courbe standard (i.e. obtenue à partir d'échantillons homogènes) permet de déterminer la teneur en HbF de chaque globule rouge de manière automatisée. On utilise pour ce faire la formule donnée par la régression linéaire associant la quantité de fluorophores par globule rouge et la TCMHbF des patients homogènes.

Par exemple, l'automatisation de la comparaison de l'intensité de fluorescence de chaque globule rouge avec une courbe standard peut être mise en oeuvre par un processeur d'un système informatique. Ce système informatique peut comporter des moyens de stockage informatique dans lequel sont stockées des instructions qui, lorsqu'elles sont exécutées par le processeur du système informatique, vont réaliser la comparaison de l'intensité de fluorescence de chaque globule rouge avec une courbe standard.

Généralement, l'homme du métier sait comment régler les paramètres du cytomètre de flux avec les données de la courbe standard de manière à obtenir directement la teneur en HbF de chaque globule rouge de l'ensemble de globules rouges analysés.

Le cytomètre de flux va ainsi pouvoir classer automatiquement les globules rouges en fonction de leur teneur en HbF.

Détermination de la quantité de globules rouges transfusés chez un patient

L'invention concerne également un procédé de détermination *in vitro* de la quantité de globules rouges transfusés chez un patient :

    a) l'obtention d'un échantillon contenant des globules rouges à partir d'un patient ;
    b) la détermination de la teneur en hémoglobine F (HbF) de chaque globule rouge d'un ensemble de globules rouges dudit échantillon selon le procédé de l'invention appliqué aux globules rouges ;
    c) l'utilisation des résultats de l'étape b) pour déterminer la quantité de globules rouges transfusés chez un patient, lesdits globules rouges transfusés ayant une teneur en HbF substantiellement égale à zéro (= 0 pg).

Selon ce procédé de l'invention, on peut classer les globules rouges en deux catégories bien distinctes. Une catégorie de globules rouges_contenant des quantités variables d'HbF (correspondant aux globules rouges du patient, quantité > 0 pg) et une catégorie de globules rouges ne contenant pas d'HbF (correspondant aux globules rouges_transfusés, quantité = 0 pg).

Dans un mode de réalisation particulier, le patient est drépanocytaire ou β-thalassémique.

Avantageusement, la quantité de globules rouges transfusés permet de déterminer un pourcentage de globules rouges transfusés par rapport aux globules rouges totaux du patient. Cela permet notamment de suivre l'évolution de la quantité de globules rouges transfusés (ou du pourcentage globules rouges transfusés) et ainsi d'aider au diagnostic dans certains cas particuliers comme par exemple la distinction entre des allo anticorps ou des auto anticorps dirigés contre des globules rouges, transfusés ou du patient.

Suivi de l'efficacité thérapeutique de la drépanocytose ou de la β-thalassémie

L'invention concerne également un procédé *in vitro* de suivi de l'efficacité thérapeutique d'un traitement de la drépanocytose ou de la β-thalassémie comprenant :

    a) l'obtention d'un échantillon contenant des globules rouges à partir d'un patient ayant subi un traitement de la drépanocytose ou de la β-thalassémie ;
    b) la détermination de la teneur en hémoglobine F (HbF) de chaque globule rouge d'un ensemble de globules rouges dudit échantillon selon le procédé de l'invention appliqué aux globules rouges ;
    c) l'utilisation des résultats de l'étape b) dans la détermination de l'efficacité thérapeutique du traitement de la drépanocytose ou de la β-thalassémie, dans lequel une efficacité thérapeutique est observée lorsqu'au moins un pourcentage prédéterminé des globules rouges de l'ensemble de globules rouges ont une teneur en HbF qui dépasse un seuil de référence et/ou lorsqu'on observe une diminution de la quantité de globules rouges ayant une teneur en HbF <2pg.

Ainsi, on peut suivre l'évolution de l'efficacité thérapeutique d'un traitement, visant à augmenter l'HbF, de la drépanocytose ou de la β-thalassémie au cours du temps, avant, pendant, et après le traitement du patient. On peut ainsi suivre l'évolution de l'efficacité thérapeutique de traitements connus, ou de nouveaux traitements. Il est donc possible d'identifier des nouveaux traitements de la drépanocytose ou de la β-thalassémie permettant d'augmenter la synthèse de l'HbF dans les globules rouges.

Dans un mode de réalisation particulier, le seuil de référence est de 2 pg ou plus, par exemple 3 pg, par exemple 4 pg, par exemple 5 pg, par exemple 6 pg, par exemple 7 pg, par exemple 7,5 pg (c'est-à-dire 7,5 pg d'HbF dans le globule rouge).

Méthode de traitement de la drépanocytose

La présente description divulgue également une méthode de traitement de la drépanocytose ou de la β-thalassémie comprenant :

a) l'obtention d'un échantillon contenant des globules rouges à partir d'un patient ;

a) la détermination de la teneur en hémoglobine F (HbF) de chaque globule rouge d'un ensemble de globules rouges dudit échantillon selon le procédé de l'invention appliqué aux globules rouges ;

b) lorsqu'au moins un pourcentage prédéterminé des globules rouges de l'ensemble de globules rouges a une teneur en HbF qui dépasse un seuil de référence, instaurer un traitement approprié chez le patient.

Par exemple, on peut évaluer la nécessité de traiter un patient ou de ne pas le traiter. On peut également choisir le traitement qui sera le plus approprié pour le patient. On peut ainsi s'assurer que le patient reçoit le traitement le plus approprié.

Dans un mode de réalisation particulier, au moins 50 % des globules rouges de l'ensemble de globules rouges ont une teneur en HbF qui dépasse le seuil de référence.

Dans un mode de réalisation particulier, le seuil de référence est de 2 pg ou plus, par exemple 3 pg, par exemple 4 pg, par exemple 5 pg, par exemple 6 pg, par exemple 7 pg, par exemple 7,5 pg (c'est-à-dire 7,5 pg d'HbF dans le globule rouge). L'article de Steinberg et al. (Blood 2014, 123, 481-485) donne des informations sur les seuils d'HbF détectables.

Le traitement approprié peut être l'hydroxyurée et/ou l'érythropoïétine.

**Légendes des figures**

Figure 1 :

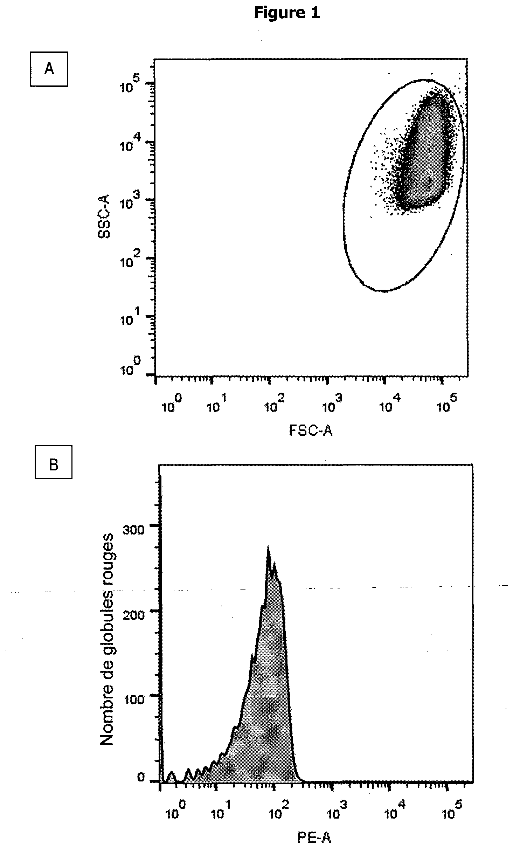

Figure 1A : Cytogramme montrant les populations de globules rouges. En abscisse : Log FSC (Forward Scatter) indiquant la morphologie des cellules. En ordonnée : Log SSC (Side Scatter) indiquant la granulosité des cellules. La sélection des globules a été effectuée sur une échelle logarithmique pour les gains des paramètres FCS et SSC et sur un échantillon de contrôle négatif.

Figure 1B : Cytogramme montrant l'histogramme de distribution de la fluorescence des globules rouges. En abscisse : Log PE-A indiquant l'intensité de fluorescence pour le fluorochrome Phycoéryhtine.

Le signal mesuré étant l'aire sous la courbe (A pour area) des variations des intensités de fluorescence de chaque cellule. En ordonnée : le nombre globule rouge.

Figure 2 :

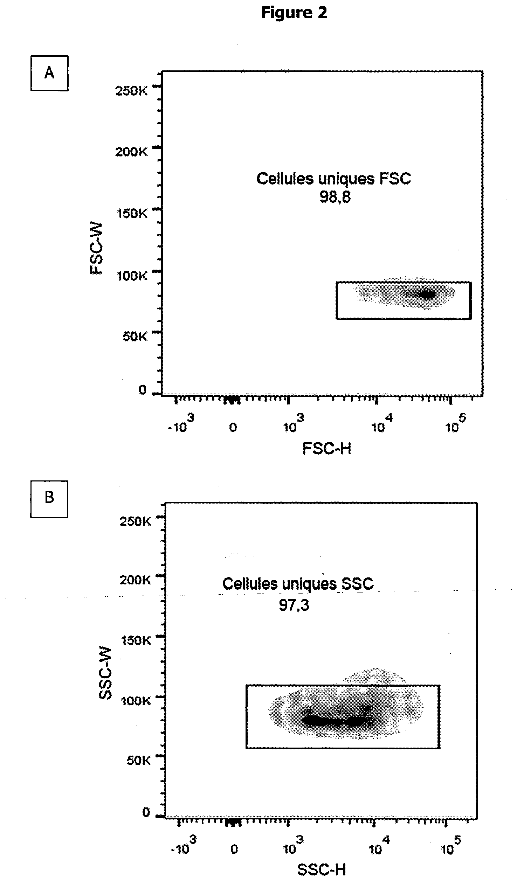

Figure 2A : Cytogramme montrant les populations de globules rouges. En abscisse : Log FSC-H (Forward Scatter- height). En ordonnée : Log FSC-W (Forward Scatter- width).

Figure 2B : Cytogramme montrant les populations de globules rouges. En abscisse : Log SSC-H (Side Scatter- height). En ordonnée : Log SSC-W (Side Scatter- width).

La sélection des singlets de globules rouges a été effectuée sur les paramètres hauteurs et largeurs du signal FSC d'abord puis sur les paramètres hauteurs et largeurs du signal SSC.

Figure 3 :

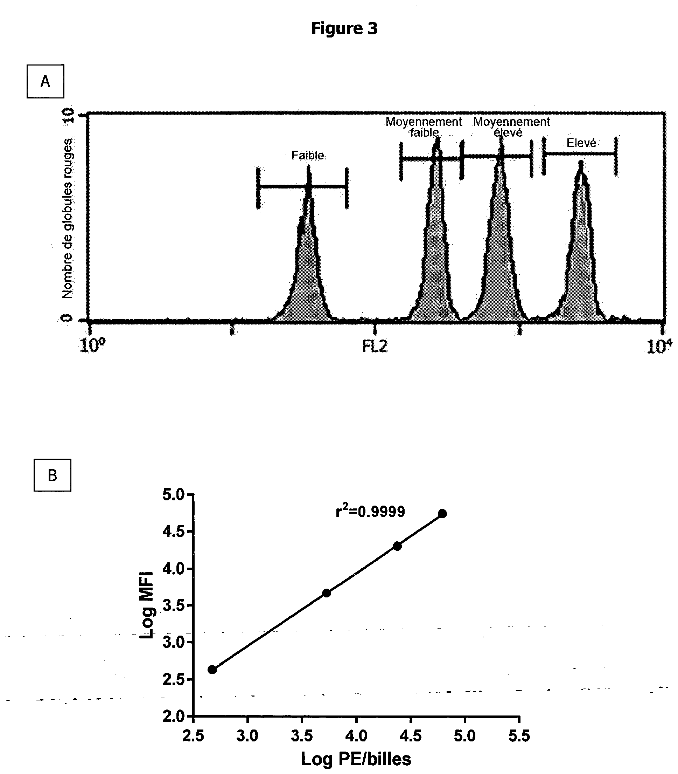

Figure 3A : Cytogramme montrant les histogrammes de distribution de la fluorescence des billes. Faible : histogramme de distribution de la fluorescence des billes couplées avec le plus faible nombre de fluorochrome (PE). Moyennement faible : histogramme de distribution de la fluorescence des billes couplées avec un nombre moyennement faible de fluorochrome (PE). Moyennement élevé : histogramme de distribution de la fluorescence des billes couplées avec un nombre moyennement élevé de fluorochrome (PE). Elevé : histogramme de distribution de la fluorescence des billes couplées avec un nombre élevé de fluorochrome (PE). En abscisse : Log FL-2 ou PE-A indiquant l'intensité de fluorescence pour le fluorochrome Phycoérythrine. En ordonnée : le nombre globule rouge.

Figure 3B : Droite de régression linéaire associant la fluorescence des 4 populations de billes (Faible, Moyennement faible, Moyennement élevé, Elevé) et le nombre de fluorochrome (PE) par bille. En abscisse : Log du nombre de fluorochrome (PE) par bille. En ordonnée : Log fluorescence des billes.

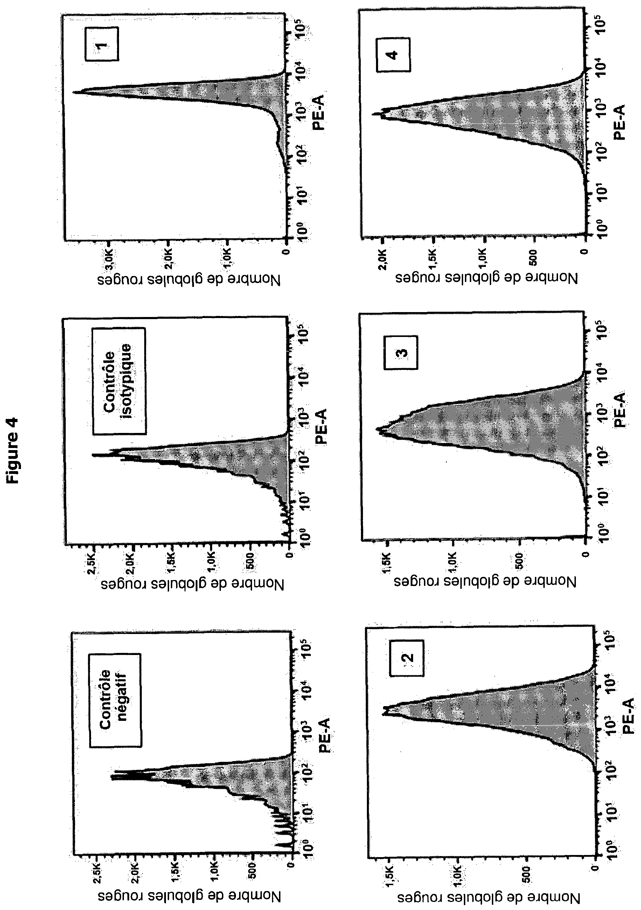

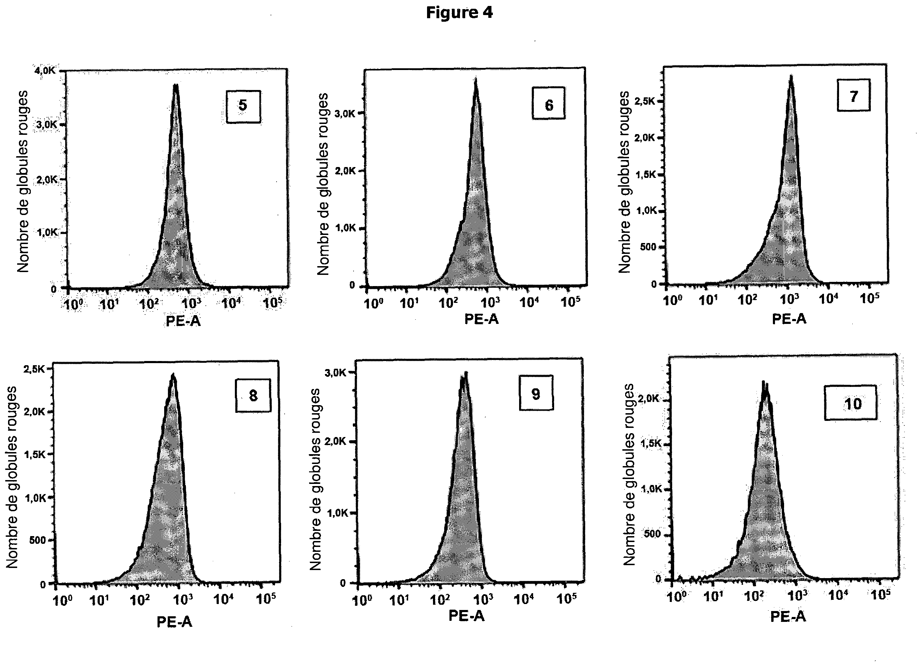

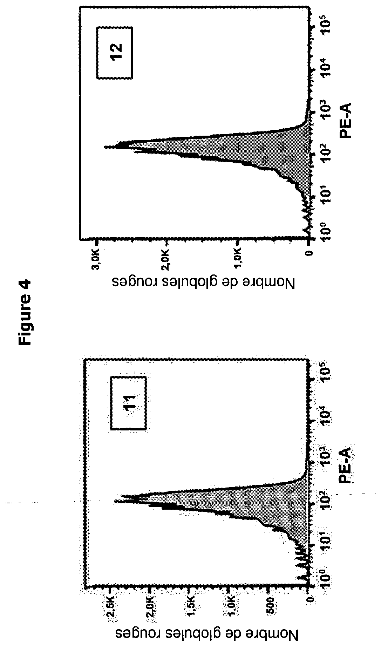

Figure 4 : Cytogrammes montrant l'histogramme de distribution de la fluorescence des globules rouges pour l'échantillon de contrôle négatif, de contrôle isotypique, et des patients 1 à 12. En abscisse : Log PE-A indiquant l'intensité de fluorescence pour le fluorochrome Phycoérythrine. En ordonnée : le nombre globule rouge.

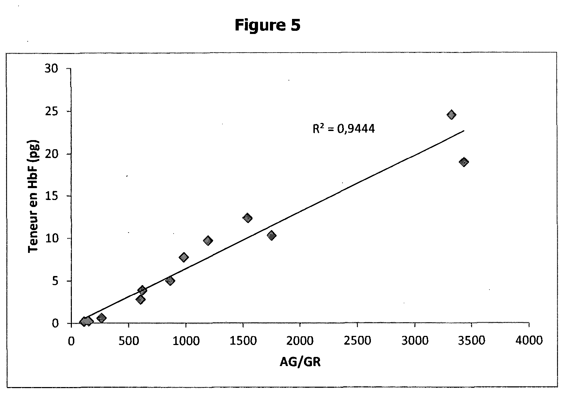

Figure 5 : Régression linéaire associant la teneur en HbF (pg) et les quantités antigéniques par globules rouges (AG/GR). En abscisse : quantités antigéniques par globules rouges (AG/GR). En ordonnée : teneur en HbF (pg).

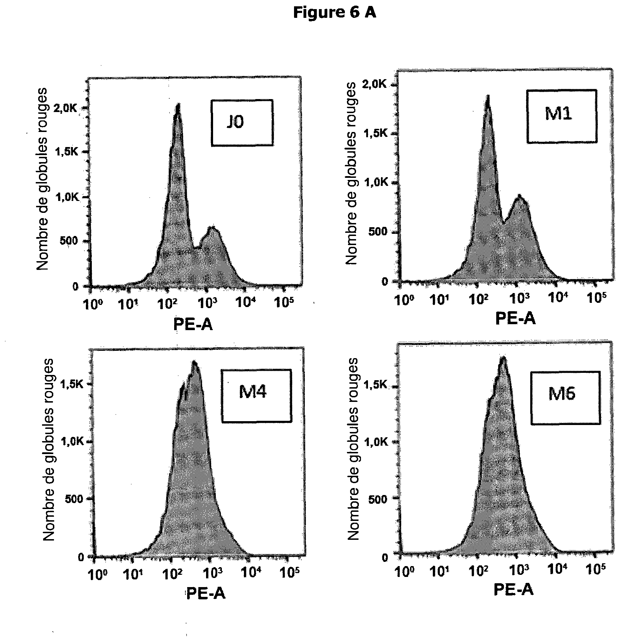

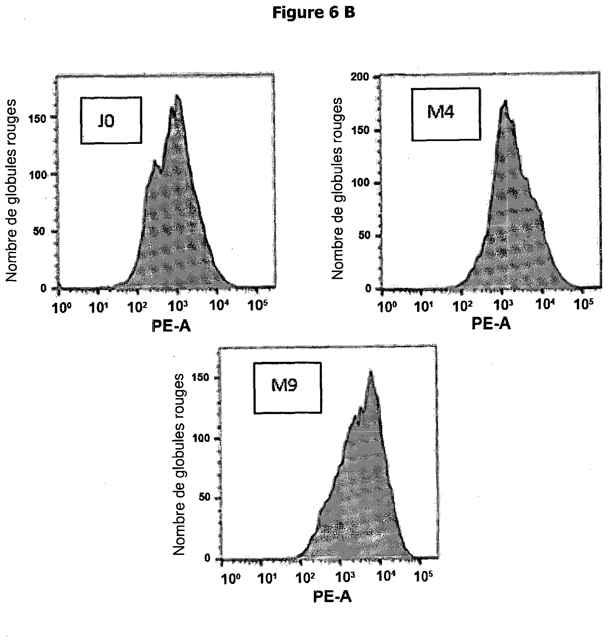

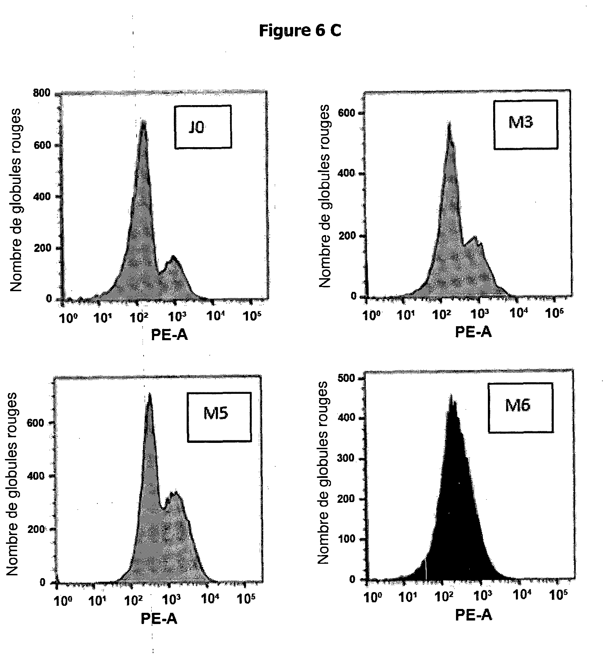

Figure 6 : Analyse de la distribution de l'HbF chez les patients A, B et C avant (30) et au cours du traitement par Hydroxyurée (M1, M3, M4, M5, M6). En abscisse : Log PE-A indiquant l'intensité de fluorescence des globules rouges pour le fluorochrome Phycoéryhtine. En ordonnée : le nombre globule rouge. Les échantillons de globules rouges ont été traités selon les mêmes procédures que celles effectuées pour les globules rouges des patients servant à la gamme de teneur en HbF.

## Exemples

Exemple 1 : Mise au point d'un procédé de détermination de la teneur en HbF de chaque globule rouge : constitution d'une courbe standard

Un groupe de patients présentant une distribution de la teneur en HbF de chaque globule rouge parfaitement homogène a été sélectionné à partir de la collection SICLOPEDIE suivie à l'Unité de Maladies Génétiques du Globule Rouge au Centre Hospitalier Universitaire Henri Mondor à Créteil. Ce groupe est composé de patients adultes (age$\geq$18 ans) présentant une persistance héréditaire en HbF (PHHF) ou une $\beta$-thalassémie intermédiaire ou mineure, et une teneur en HbF de chacun de leurs globules rouges homogène et constante dans le temps. La teneur en HbF des globules rouges totaux des patients a été déterminée, dans le cadre du suivi des patients, par une mesure régulière du %HbF (par HPLC) et de la teneur moyenne en HbF par globule rouge (TCMHbF). Les patients présentant un syndrome drépanocytaire majeur (SS, S$\beta$-Thalassémie ou SC) ou un trait drépanocytaire (AS) ainsi que les patients traités par un inducteur de l'HbF (Hydroxyurée), les patients ayant été transfusés durant les 3 derniers mois avant l'inclusion et les patientes présentant une grossesse n'ont pas été inclus.

En accord avec la déclaration d'Helsinki sur les principes éthiques applicables à la recherche médicale impliquant des êtres humains (Association Médicale Mondiale, Texte en vigueur 2008, paragraphe 24), tous les patients sélectionnés ont été informés des risques et des bénéfices de cette étude et ont fourni un consentement écrit avant l'inclusion. Conformément aux législations en vigueur (articles L.1121-3 et R.5121-13 du code de la santé publique), les informations relatives aux patients ont été protégées par l'anonymat afin d'assurer la confidentialité pendant toute la durée de l'étude.

Ce protocole de recherche a obtenu l'avis favorable du Comité de Protection des Personnes Île-de-France IV sis à l'Hôpital Saint-Louis (n° IRB 00003835).

En résumé, 20mL de sang ont été prélevés chez les patients sélectionnés et collectés dans 5 tubes contenant de l'EDTA. Les échantillons ont été traités dans les 24h suivant les prélèvements : détermination du pourcentage moyen en HbF (en utilisant l'HPLC), hémogramme sanguin, et analyse en cytométrie en flux puis congélation des échantillons restant à -80°C pour les analyses ultérieures.

Le protocole détaillé est expliqué ci-dessous.

## 1. Pré-traitement des échantillons

20mL de sang ont été prélevés chez les patients sélectionnés et collectés dans 5 tubes contenant de l'EDTA. Les globules rouges ont été récupérés après fractionnement du sang total par centrifugation à 800G pendant 10 minutes à température ambiante. Environ 8mL de culot globulaire ont été collectés puis lavés dans 10mL de tampon phosphate (DPBS 1X Gibco by life technologies Cat 14190-094, Life Technologies SAS, Saint Aubin, France) dans un tube en polypropylène de 50mL. Après homogénéisation, les suspensions globulaires ont été centrifugées à 1200G pendant 5 minutes à température ambiante. Le culot globulaire a été récupéré après élimination du surnageant et la procédure de lavage a été effectuée 3 fois.

Une première mesure de la teneur en HbF (%HbF par HPLC x TCMH) a été réalisée sur environ 1mL d'échantillon frais tandis que 7ml de culot globulaire ont été conservés à - 80°C par fractions aliquotes de 200$\mu$L dans des cryotubes (Nalgene Cat 479-6841) en utilisant du Glycérol (B Braun formula N°569) comme agent cryoconservateur. Pour cela, environ 57.7% (V/V) de glycérol ont été ajouté dans les culots globulaires en 2 étapes. En effet, afin de prévenir la formation de précipités, 1/3 du volume de glycérol a été ajouté dans un premier temps, dans le culot globulaire par gouttes en agitant le cryotube suivi d'une incubation de 10 minutes à température ambiante, le volume de glycérol a été par la suite complété par les 2/3 restants. Avec cette procédure, les globules rouges peuvent être conservés pendant plusieurs mois, voire des années, tout en limitant leur lyse.

La décongélation consiste en une incubation des globules rouges dans un bain à 37°C ou en une agitation rapide dans la main suivies d'une série de lavage en utilisant 2 solutions de NaCl en concentrations décroissantes afin d'éliminer le glycérol et de conserver l'isotonicité. Pour cela, 75$\mu$L de solution de déglycérolysation (NaCl 12%, B Braun formula N°570 Melsungen, Allemagne) ont été ajoutés dans un premier temps par gouttes en homogénéisant les suspensions globulaires puis les suspensions ont été incubées à température ambiante pendant 10 minutes. Par la suite, 125$\mu$L de NaCl à 0.9% glucosé ont été ajoutés et les suspensions ont été incubées pendant 5 minutes à température ambiante. Environ 4*125$\mu$L de NaCl à 0.9% glucosé ont été ajoutés en respectant une incubation de 5 minutes entre chaque volume de NaCl à 0.9% glucosé. Les suspensions globulaires ont été transférées dans des tubes Eppendorf de 2mL puis fractionnées par une centrifugation douce à 300G pendant 10 minutes à température ambiante en appliquant une décélération égale à 5. Après élimination du surnageant, 4*125$\mu$L de NaCl à 0.9% glucosé ont été ajoutés en respectant une incubation de 5 minutes entre chaque volume de NaCl à 0.9% glucosé puis les suspensions ont été centrifugées comme précédemment. Afin d'obtenir un culot sec de globules rouges, 500pL de NaCl à 0.9% glucosé ont été ajoutés en une fois suivi d'une centrifugation à 800G pendant 10 minutes à température ambiante. A la fin de la dernière centrifugation, les échantillons globulaires peuvent être ramenés à l'hématocrite voulue en complétant par une solution tampon.

## 2. Détermination de la teneur en HbF de chaque globule rouge

La détermination de la teneur en HbF de chaque globule rouge des échantillons de patients sélectionnés a été réalisée aussi bien dans les échantillons globulaires frais que dans les échantillons décongelés à partir des 3 méthodes suivantes :

- la chromatographie liquide à haute performance (HPLC) pour déterminer le pourcentage moyen en HbF sur l'ensemble des globules rouges ;
- l'hémogramme sanguin (incluant la TCMH moyenne) ; et
- la cytométrie en flux.

### 2.1. Détermination du pourcentage moyen en HbF par HPLC

La détermination du pourcentage moyen en HbF par HPLC dans les suspensions globulaires a été effectuée sur un système Variant II (Cat : 2702000 Hemoglobin Testing System, Bio-Rad Laboratories, Marnes-la-Coquette, France). Il s'agit d'une chromatographie liquide par exclusion d'ions permettant de séparer 3 types d'hémoglobines : HbF, HbA et HbA2 grâce à la méthode V2_B-THAL_DU. Le système est composé d'une phase stationnaire composée de résine sur laquelle sont fixés des groupements carboxyles chargés négativement et d'une phase mobile composée de deux solutions tampon Bis/Tris Phosphate présentant une faible et une importante force ionique respectivement ainsi que d'un détecteur photométrique émettant 2 longueurs d'ondes différentes : 690 nm et 415 nm pour la détection du control négatif et des échantillons respectivement. Ce système permet de quantifier les 3 hémoglobines à partir du sang total ou d'une suspension globulaire de volume minimal de 500$\mu$l. Pour un échantillon inférieur à 500pl, une dilution à 1:200 (v/v) avec un réactif de dilution a été effectuée avant de procéder à l'analyse. Les analytes ont été séparés en fonction de leur interaction ionique avec les groupements carboxyl sur une durée totale de 6 minutes. L'HbF est éluée au bout de 0,5 minutes environ. L'acquisition des données a été effectuée sur un programme CDM 5.2 afin de déterminer les pourcentages moyens de chaque type d'Hb. Le pourcentage moyen en HbF s'obtient selon la relation suivante :

$$\%HbF= (ASC\ HbF/\ ASC\ total\ Hb)*100$$

où %HbF représente le pourcentage moyen en HbF sur l'ensemble de la population érythrocytaire ;

ASC HbF représente l'aire sous la courbe de l'HbF sur le chromatogramme ;

ASC total représente la somme des aires sous les courbes des 3 différents types d'Hb, correspondant chez ces patients à HbA, HbA2 et HbF.

*2.2. Détermination de la teneur moyenne en hémoglobine par globule rouge (TCMH)*

La teneur moyenne en hémoglobine par globule rouge (ou teneur corpusculaire moyenne en hémoglobine ou TCMH) constitue une constante globulaire obtenue par numération et formule sanguine ou hémogramme sanguin. La TCMH est déterminée par calcul en divisant la concentration d'hémoglobine exprimée par litre de sang par le nombre de globules rouges par litre de sang.

Cette analyse a été effectuée sur une automate de numération Horiba ABX Micros ES 60 (HORIBA Medical, Montpellier, France) sur un volume minimal d'échantillon à 500$\mu$L présentant un hématocrite à environ 50%.

*2.3. Détermination de la teneur moyenne en HbF par globule rouge (TCMHbF)*

La détermination de 2 paramètres moyens (%HbF et TCMH) obtenues par HPLC et par l'hémogramme sanguin a permis de calculer la teneur moyenne en HbF par globule rouge ou TCMHbF selon la relation suivante : TCMHbF = (%HbF * TCMH)/100

où TCMHbF (pg) représente la teneur moyenne en HbF par globule rouge ;

%HbF représente le pourcentage moyen en l'HbF déterminé par HPLC (voir 2.1) ;

TCMH représente la teneur moyenne en Hb par globule rouge fournie par l'automate de numération (voir 2.2).

3. Détermination de la fluorescence des globules rouges marqués par un anticorps anti HbF en cytométrie en flux

*3.1. Instrumentation*

L'analyse de la teneur en HbF de chaque globule rouge a été effectuée sur un cytomètre BD FACS Canto II à 8 couleurs (Cat 338960, BD Biosciences, Le Pont de Claix, France) associé avec le logiciel d'acquisition des données DIVA®.

La cytométrie en flux ou FACS (fluorescence-activated cell sorting) est une technique permettant d'analyser individuellement les propriétés (taille, granulosité et fluorescence) des particules (billes ou cellules) en suspension dans un système en flux.

C'est une méthode immunologique basée sur la technique d'immunophénotypage qui consiste en une détection antigénique grâce à la reconnaissance par un anticorps spécifique qui est le plus souvent conjugué à une molécule fluorescente. Le signal mesuré est principalement l'intensité de fluorescence qui est proportionnelle à la quantité d'antigènes détectés par l'anticorps spécifique.

L'acquisition des données au cytomètre requiert un traitement au préalable des échantillons qui consiste en la fixation et perméabilisation de la membrane cellulaire suivie du marquage intracellulaire avec un anticorps spécifique de l'HbF conjuguée à un fluorochrome.

*3.2. Fixation et perméabilisation de la membrane des globules rouges*

La fixation et la perméabilisation de la membrane des GR ont été effectuées en utilisant les réactifs d'un Kit de détection des cellules F appelé « Fetal Cell Count™ kit » (Cat IQP-349, IQ Products, Groningen, Netherlands).

Afin de fixer la membrane globulaire, 5$\mu$L de culot globulaire lavé (frais ou décongelé) ont été ajoutés et homogénéisés dans 100$\mu$L de réactif de fixation ou réactif A contenant un agent conservateur ou azide de sodium (Fetal Cell Count™ kit, Cat IQP-349). Par la suite 100 $\mu$L de solution tamponnée de formaldéhyde ou solution de fixation B (Fetal Cell Count™ kit, Cat IQP-349) ont été ajoutés à la suspension globulaire. La suspension a été homogénéisée par agitation au vortex puis incubée à température ambiante pendant exactement 30 minutes. La suspension globulaire a été homogénéisée délicatement toutes les 10 minutes. Les GR ont été par la suite lavés dans 2 ml de tampon PBS 1X contenant de l'héparine (réactif D Fetal Cell Count™ kit, Cat IQP-349) suivi d'une centrifugation à 300G pendant 3 minutes à température ambiante. Le culot globulaire lavé a été ramené en suspension en ajoutant 100µL de tampon PBS 1X (réactif D Fetal Cell Count™ kit, Cat IQP-349).

Afin de perméabiliser la membrane globulaire, 100 µL de solution de Dodecyl Sulfate de Sodium, réactif de perméabilisation (réactif C Fetal Cell Count™ kit, Cat IQP-349) ont été ajoutés dans la suspension des GR fixés. Les échantillons ont été incubés pendant exactement 3 minutes à température ambiante. Les GR ont été lavés comme précédemment. Le lavage a été effectué 2 fois. Après suppression du surnageant, les culots globulaires ont été ramenés en suspension dans 1mL de tampon PBS 1X (réactif D Fetal Cell CountTM kit, Cat IQP-349).

*3.3. Marquage intracellulaire des globules rouges fixés et perméabilisés*

Un anticorps monoclonal de souris de clone IgG1 Kappa dirigé spécifiquement contre la chaîne gamma de l'HbF humaine et conjugué avec la phycoérythrine (PE) (réactif F Fetal Cell CountTM kit, Cat IQP-349, IQ Products) a été utilisé pour déterminer la teneur en HbF de chaque globule rouge préalablement fixé et perméabilisé. Pour cela, 50µL de solution d'anticorps ont été déposés dans 50µL de suspension obtenue au 3.2. La phycoérythrine, par ses propriétés conformationnelles (encombrement stérique) présente l'avantage d'avoir un ratio ratio PE : anticorps proche de 1.

Un échantillon non marqué avec l'anticorps a été utilisé comme control négatif tandis qu'un contrôle isotypique a été préparé en ajoutant 20µL d'IgG1 Kappa de souris couplé avec PE (Cat : 555749, BD Pharmingen™, Le Pont De Claix, France) dans 50 µL de suspension obtenue au 3.2 afin de vérifier la spécificité de fixation de l'anticorps anti HbF utilisé.

Les échantillons marqués, non marqué ainsi que le control isotypique ont été incubés pendant 15 minutes à l'abri de la lumière et à température ambiante. Les GR ont été par la suite lavés dans 2 ml de tampon PBS 1X (réactif D Fetal Cell Count™ kit, Cat IQP-349) suivi d'une centrifugation à 300G pendant 3 minutes à température ambiante. Les culots globulaires lavés ont été ramenés en suspension en ajoutant 500µL de tampon PBS 1X (réactif D Fetal Cell Count™ kit, Cat IQP-349). Les globules rouges ont été analysés au cytomètre dans les minutes suivant leur pré-traitement.

*3.4. Analyse des échantillons au cytomètre*

Au cours de l'analyse, les échantillons ont été placés dans une enceinte froide (glace) et à l'abri de la lumière afin d'éviter les pertes de fluorescences. Avant l'acquisition des données, le cytomètre est préalablement calibré selon les recommandations du fournisseur.

L'échantillon non marqué a été utilisé pour déterminer les voltages (ou PMT) de chaque paramètre : FSC (Forward Scatter), SSC (Side Scatter) et fluorescence (Tableau 1). La sélection des populations de globules rouges a été basée sur les critères de taille ou FSC et de granulosité ou SSC sur le cytogramme Log FSC-A vs Log SSC-A en éliminant les débris et les bruits de fond. Le contrôle négatif a été déterminé sur le signal généré par l'échantillon non marqué en fluorescence (figure 1). Les échelles logarithmiques ont été privilégiées pour les gains FSC et SSC.

Tableau 1 : Réglage du cytomètre obtenu avec l'échantillon de globules rouges non marqués

| Paramètre | Voltage | Log | A | H | W |
|-----------|---------|-----|-----|-----|-----|
| **FSC** | 341 | √ | √ | √ | √ |
| **SSC** | 328 | √ | √ | √ | √ |
| **PE** | 400 | √ | √ | √ | √ |

L'exclusion des doublets de globules rouges (GR) a été effectuée avec une faible vitesse de flux en sélectionnant les populations de GR sur les cytogrammes FSC-W vs FSC-H puis SSC-W vs CCS-H selon la figure 2.

Un nombre minimum de 100000 évènements ou GR ont été collectés afin d'améliorer la précision de l'analyse et d'enregistrer un nombre suffisant de GR après l'exclusion des doublets. Pour chaque échantillon, les statistiques ont été générées par le logiciel d'acquisition (Diva®) avec le nombre d'évènements enregistrés pour chaque population de GR sélectionnée. L'intensité de fluorescence (MFI) est donnée en moyenne géométrique et moyenne arithmétique.

4. Détermination de la quantité d'HbF marquée concentration antigénique par globule rouge ou AG/GR

Afin de déterminer la quantité d'HbF marquée par globule rouge, l'intensité de fluorescence obtenue au cytomètre de flux a été transformée en quantité d'HbF marquée par globule rouge (AG/GR) à l'aide des billes calibrées contenues dans un Kit de quantification de la fluorescence de la phycoérythrine (Becton Dickinson QuantiBRITE PE, Cat : 340495, BD Biosciences, Le Pont De Claix, France). Ces billes sont conjuguées à la phycoérythrine de manière à fournir 4 niveaux de fluorescence (faible ou Low, moyennement faible ou MedLow, moyennement élevée ou MedHigh et élevée ou High) correspondant à un nombre de molécules de phycoérythrine par bille (figures 3A). Avant l'acquisition des données de fluorescence au cytomètre, les billes ont été reconstituées dans $500\mu L$ de solution tampon de marquage composée de PBS 1X /0.02% NAN3 (v/v) (Cat : 296028 ChemCruz™ Biochemicals, Santa Cruz Biotechnology, Germany) supplémenté avec 1% de BSA (Cat : A3803, Sigma, Saint-Quentin Fallavier France). Au minimum 10000 billes ont été collectées avec les mêmes réglages utilisés pour les échantillons analysés au point 3.4. Les données acquises au cytomètre ont permi de tracer une droite de calibration associant Log fluorescence (Log MFI) et Log PE/bille (figure 3B). En appliquant cette dernière sur la fluorescence des GR marqués par l'anticorps anti HbF, on peut déduire la quantité de molécules fluorescentes par globule rouge. En utilisant le ratio PE : anticorps, le nombre de molécules fluorescentes par globule rouge peut être converti en nombre d'anticorps liés par globule rouge, c'est-à-dire en nombre de molécule antigénique (i.e. HbF) par globule rouge (AG/GR).

5. Détermination de la teneur en HbF (pg) de chaque GR à partir de AG/GR et de la TCMHbF

Les données enregistrées par le logiciel d'acquisition du cytomètre de flux (Diva®) ont été traitées sur FlowJo V10 (Cat 130-099-429 Miltenyi Biotec, Paris, France). Il s'agit d'un programme d'analyse et de visualisation des données de cytométrie exportées sous format « flow cytometry standard » ou fcs. Le logiciel FlowJo a permis de générer les valeurs de chaque paramètre (FSC, SSC, Fluorescence) pour chaque événement.

Pour chaque échantillon de GR collecté pour cette étude, la distribution de l'HbF marquée par l'anticorps est représentée par un histogramme associant le nombre de globules rouges et le Log de fluorescence.

La teneur moyenne en HbF par globule rouge (TCMHbF) de chaque échantillon de patients sélectionnés obtenue par la combinaison de l'HPLC et de la TCMH a été associée à la moyenne de fluorescence transformée en AG/GR grâce aux billes de quantification.

Afin de déterminer la teneur en HbF (en pg) de chaque globule rouge, une courbe standard a été construite à partir des TCMHbF et des valeurs AG/GR donnant ainsi une gamme de TCMHbF connues associées à la fluorescence (figure non présentée).

Exemple 2 : validation de la méthode de détermination de la teneur teneur en HbF de chaque globule rouge

1. Caractéristiques des patients inclus

Une cohorte de 12 patients a été sélectionnée afin de constituer une gamme de teneur en HbF, le critère de sélection de ces patients était la confirmation d'une distribution homogène, Log Normale, de leur HbF. Les données issues de ces patients ont été utilisées pour construire une courbe standard (cf Exemple 1 point 5) servant à déterminer la teneur en HbF de chaque globule rouge. Le tableau 2 résume les données démographiques et les caractéristiques génétiques de ces patients.

Tableau 2 : Données démographiques et biologiques des patients inclus pour construire la courbe de standard

| Patients | Age à l'inclusion (années) | Genre | Caractéristiques génétiques | HbF (%) | TCMH (pg) | TCMHbF (P9) | VGM ($\mu m^3$) |
|---|---|---|---|---|---|---|---|
| 1 | 18 | Homme | β-thalassémie | 100 | 24,5 | 24,5 | 73 |
| 2 | 62 | Homme | β-thalassémie | 69,6 | 27,2 | 18,93 | 91 |
| 3 | 50 | Homme | β-thalassémie | 61,5 | 20,1 | 12,36 | 64 |
| 4 | 46 | Homme | β-thalassémie | 53,2 | 19,3 | 10,27 | 63 |
| 5 | 50 | Femme | HPFH2 Ghanéenne | 34,8 0 | 28 | 9,74 | 81 |
| 6 | 39 | Femme | β-thalassémie | 27,9 | 28 | 7,76 | 81 |
| 7 | 39 | Homme | β-thalassémie | 21 | 23,8 | 5 | 72 |
| 8 | 43 | Femme | β-thalassémie | 16,4 | 23,6 | 3,87 | 71 |
| 9 | 37 | Homme | β-thalassémie | 14,9 | 18,5 | 2,76 | 62 |
| 10 | 60 | Femme | β-thalassémie | 2,7 | 23,4 | 3,11 | 67 |
| 11 | 58 | Homme | β-thalassémie | 0,8 | 19,4 | 0,15 | 64 |

(suite)

| Patients | Age à l'inclusion (années) | Genre | Caractéristiques génétiques | HbF (%) | TCMH (pg) | TCMHbF (P9) | VGM ($\mu m^3$) |
|---|---|---|---|---|---|---|---|
| 12 | 23 | Homme | β-thalassémie | 0,8 | 27,8 | 0,22 | 83 |

Une distribution homogène des fluorescences des globules rouges et donc de la teneur en HbF a été vérifiée sur l'ensemble des échantillons recueillis (figure 4). La figure 4 montre en échelle semi-logarithmique la distribution Log normale de la teneur en HbF par GR des 12 patients de la gamme.

2. Test de reproductibilité des différentes mesures

Une étude de reproductibilité a été effectuée en testant les variabilités sur les mesures suivantes :

- TCMHbF sur les globules rouges frais et après une série de décongélation
- MFI obtenues avec les billes QuantiBRITE PE et les GR
- AG/GR après une série de décongélation

Aucune différence significative n'a été observée sur les TCMHbF (pg) obtenues sur des globules rouges frais et après 12 décongélations (n=13, p>0 ,9999) ni sur les AG/GR mesurées après 11 décongélations (p= 0,73). De même, l'analyse de la variabilité des données obtenues sur les billes de quantifications n'a révélée aucune différence significative (n=11, p=0,99).

3. Méthode de mesure de la teneur en HbF dans chaque globule rouge

La gamme de TCMHbF obtenue chez les patients présentant une distribution des teneurs en HbF par globule rouge homogène a été associée aux intensités de fluorescences puis aux quantités antigéniques par globule rouge à l'aide des billes de calibration. Une courbe standard (ou droite de régression) a été déterminée à partir des moyennes des AG/GR obtenues après 11 décongélations et les TCMHbF de chaque patient (figure 5).
Un coefficient de corrélation à 97.18% ($r^2$=0.9444) a été obtenu entre les TCMHbF et les moyennes des quantités antigéniques d'HbF (marquée par l'anticorps fluorescent) par globule rouge, pour les différents patients. Cette courbe standard permet de déterminer la teneur en HbF par GR pour une valeur de fluorescence donnée. La précision des mesures est fournie par un intervalle de confiance à 95% déterminée à partir de la courbe standard « moyenne », des écart-types entre chaque régression et du nombre de modèle de régression ayant servi à calculer la moyenne.

Exemple 3 : Etude de la distribution de l'HbF au cours du traitement par Hydroxyurée

1. Patients

Une étude prospective, longitudinale et monocentrique a été menée sur une cohorte de 29 patients drépanocytaires adultes (age≥18ans) présentant une mutation homozygote SS, débutant un traitement par hydroxyurée, suivis régulièrement au Centre de référence des syndromes drépanocytaires majeurs au Centre Hospitalier Universitaire Henri Mondor à Créteil. Les patients ayant eu une crise nécessitant une hospitalisation et/ou un programme d'échange transfusionnel durant les 3 derniers mois avant l'inclusion ainsi que les patientes présentant une grossesse en cours ou ayant un désir de grossesse ont été exclus de l'étude.
Les patients présentant les critères d'éligibilité ont été suivis à différents temps avant et pendant le traitement par hydroxyurée : 30 (avant le début du traitement par hydroxyurée), à 15 jours, 1 mois, 3 mois, 4 mois et 6 mois ou plus après la mise en place du traitement par hydroxyurée (J15, M1, M3, M4 et ≥M6 respectivement).
En accord avec la déclaration d'Helsinki sur les principes éthiques applicables à la recherche médicale impliquant des êtres humains (Association Médicale Mondiale, Texte en vigueur 2008, paragraphe 24), tous les patients recrutés ont été informés des risques et des bénéfices de cette étude et ont fourni un consentement écrit avant l'inclusion. Conformément aux législations en vigueur (articles L.1121-3 et R.5121-13 du code de la santé publique), les informations relatives aux patients ont été protégées par l'anonymat afin d'assurer la confidentialité pendant toute la durée de l'étude.
Ce protocole de recherche a obtenu l'avis favorable du Comité de Protection des Personnes Île-de-France IV sis à l'Hôpital Saint-Louis (n° IRB 00003835).

2. Méthodes

Lors de chaque visite au centre (J0, J15, M1, M3, M4 et ≥M6), environ 16mL de sang ont été prélevés et collectés dans 4 tubes contenant de l'EDTA.

*2.1. Prétraitement des échantillons globulaires*

Les échantillons ont été traités dans les 24h suivant les prélèvements. Il s'agit de la séparation des globules rouges en fonction de leur densité et de la cryoconservation des fractions globulaires et des globules rouges totaux ou non fractionnés.

3. Analyse des échantillons globulaires

Les différentes mesures réalisées sur chaque fraction globulaire ou sur les GR totaux ont été composées de :

- la numération et formule sanguine ou hémogramme sanguin par une automate de numération ;
- la mesure du pourcentage moyen en HbF (%HbF) par HPLC ; et
- la mesure par cytométrie de flux de l'intensité de fluorescence de chaque GR marqués avec un anticorps dirigé contre l'HbF conformément au protocole détaillé à l'Exemple 1.

*3.1. Numération et formule sanguine, mesure du pourcentage moyen en HbF (%HbF) par HPLC*

Ces analyses ont été effectuées sur les GR frais et après décongélation selon les mêmes techniques que celles décrites à l'Exemple 1.

*3.2. Mesure de la teneur en l'HbF de chaque GR par cytométrie en flux*

Cette analyse a été effectuée après décongélation des GR conservés à -80°C selon la technique décrite dans l'Exemple 1.

La détermination de la teneur en HbF de chaque GR a été réalisée en utilisant la courbe standard décrite dans l'Exemple 1 à partir des intensités de fluorescence de chaque globule rouge transformées en AG/GR par les billes QuantiBRITE PE. Les mesures ont été effectuées à chaque stade du traitement (J0, J15, M1, M3, M4 et ≥M6) dans les différentes fractions globulaires : globules rouges non denses, denses et totaux.

4. Analyses statistiques

Chez les patients SS sous hydroxyurée, les pourcentages de GR contenant des teneurs en HbF regroupées en classes définies arbitrairement (0 à 2 pg, 2 à 4 pg, 4 à 6 pg etc...) ont été comparés au cours du suivi longitudinal afin de déterminer les variations de la distribution de l'HbF avant et au cours du traitement par hydroxyurée. Les comparaisons entre chaque stade du traitement ont été effectuées au moyen d'une ANOVA mesure répétée entre J0, J15-M3 et ≥M6 dans les globules rouges totaux.

Les analyses statistiques ont été effectuées à l'aide du logiciel GraphPad Prism®Version 6 (RITME Informatique Paris France).

5. Résultats

*5.1. Données démographiques et indications de l'Hydroxyurée*

Au total, 29 patients SS adultes ont été inclus. Sur l'ensemble de la cohorte, 10 patients ont été suivis entre 30 et ≥M6, les échantillons de GR d'un patient ont hémolysé, 9 patients ont été analysés.

Les données démographiques des patients inclus sont résumées dans le tableau 3.

Tableau 3 : Données démographiques des patients SS sous hydroxyurée

|   | Hommes | Femmes |
|---|--------|--------|
| **N** | 13 | 16 |

(suite)

|  | Hommes | Femmes |
|---|---|---|
| **Age** | 33 [28,50 ; 45] | 36 [31 ; 44,25] |
| *Age exprimé en Médiane [25eme percentile ; 75eme percentile]* | | |

L'hydroxyurée a été administrée avec une dose moyenne de 15mg/Kg/jour pour les indications suivantes : prévention des CVO, atteintes viscérales chroniques, ulcères de jambes, néphropathie drépanocytaire, hémolyse importante avec anémie, priapisme, AVC.

*3.2. Données biologiques*

Le pourcentage moyen en HbF (%HbF) augmente d'environ 3 fois après 6 mois de traitement par hydroxyurée. Les données sont présentées dans le tableau 4.

Tableau 4: Variation des %HbF au cours du traitement par Hydroxyurée

|  | J0 | J15-M1 | M3-M4 | M6 | p (J0 et M6) |
|---|---|---|---|---|---|
| **Globules rouges totaux** | 2,65 [1,8-6,35] | 4,9 [1,85-8,4] | 7 [2,4-17,80] | 10,05 [3,33- 19,13] | *0,03* |
| *Données exprimées en Médiane [25eme percentile ; 75eme percentile]* | | | | | |

Une augmentation significative du Volume globulaire moyen (VGM) et de la teneur moyenne en hémoglobine par globule rouge (TCMH) a été observée après 6 mois de traitement par Hydroxyurée indiquant une bonne conformité des patients et l'efficacité du traitement malgré le faible effectif. Le pourcentage de globules rouges denses présente une tendance à diminuer, cependant cette diminution n'est pas significative. De même pour la concentration corpusculaire moyenne en hémoglobine (CCMH), les leucocytes et les plaquettes, aucune variation significative n'a été observée. Les résultats sont présentés dans le tableau 5.

Tableau 5 : Données biologiques avant et au cours du traitement par hydroxyurée

|  | J0 | M6 | p |
|---|---|---|---|
| **Hémoglobine (g/dl)** | 8 [6,65-9,05] | 9,35 [7,30-10,10] | 0,2 |
| **Leucocytes (Giga/L)** | 10,70 [9,45-12,20] | 9 [5,35-11,58] | 0,11 |
| **VGM (fento litre)** | 87 [83-95] | 100,50 [87,75-115] | *0,03* |
| **CCMH (g/dl)** | 32,60 [32,05-35,10] | 34,40 [32,43-35,70] | 0,08 |
| **TCMH (pg)** | 29,70 [25,30-32,85] | 33,80 [30,70-39,65] | *0,008* |
| **Plaquettes (giga/L)** | 359 [288-548] | 318 [257-438.80] | 0.18 |
| *Données exprimées en Médiane [25eme percentile ; 75eme percentile]* | | | |

*5.3. Distribution de l'HbF au cours du traitement par Hydroxyurée*

L'analyse qualitative par cytométrie de flux de la réponse au traitement par hydroxyurée montre une distribution hétérogène de l'HbF avant le début du traitement chez 8 patients. Après 6 mois de traitement par Hydroxyurée, l'HbF suit une distribution homogène chez 6 patients. La distribution de l'HbF devient homogène à partir de M2 chez 1 patient et après ≥M4 chez 5 patients. La figure 6 illustre les variations de la distribution de l'HbF au cours du traitement par Hydroxyurée dans les globules rouges totaux.

La distribution de l'HbF a été analysée quantitativement grâce à la méthode décrite dans l'Etude 1. Le nombre de globules rouges par intervalle de teneur en HbF (pg) a été déterminé pour chaque patient L'analyse de la répartition des globules rouges au cours du temps sous Hydroxyurée a été effectuée chez 9 patients. Les résultats montrent une variation significative des globules rouges présentant les plus faibles teneurs en HbF (0 et 2pg) avec une diminution d'environ 25% entre J0 et M6. Les globules rouges présentant une teneur en HbF supérieure à 20g augmentent de façon importante, jusqu'à 3 fois, après 6 mois de traitement par hydroxyurée, toutefois cette variation est non significative. Les globules rouges présentant des teneurs moyennement élevées présentent des variations beaucoup plus faibles.

Tableau 6 : Répartition des globules rouges par intervalle de teneur en HbF sous traitement par hydroxyurée

| HbF (pg) | 30 (% GR) | J15 (% | ≥M6 (% GR) | p |
|---|---|---|---|---|
| [0; 2[ | 71,13 (23,16) | 65,63 (25,13) | 53,17 (30,63) | **0,0083** |
| [2 ; 4[ | 9,14 (7,44) | 11,32 (6,80) | 15,05 (10,11) | 0,1868 |
| [4 ; 6[ | 4,54 (3,86) | 4,88 (2,90) | 6,70 (4,39) | 0,2226 |
| [6 ; 8[ | 3,16 (2,92) | 3,42 (2,52) | 3,92 (2,44) | 0,5186 |
| [8 ; 10[ | 2,42 (2,32) | 2,65 (2,18) | 2,72 (1,83) | 0,6932 |
| [10 ; 15[ | 1,94 (1,84) | 2,41 (2,73) | 2,55 (2,57) | 0,3926 |
| [15 ; 20[ | 3,66 (3,35) | 4,42 (4,27) | 4,29 (3,42) | 0,474 |
| ≥20 | 4,01 (4,35) | 5,27 (8,56) | 11,60 (18,64) | 0,1626 |

*Les données sont exprimées en moyenne (écart-type)*

La mesure des intensités de fluorescence de chaque globule rouge par cytométrie de flux a permis de classer chaque globule rouge en fonction de leur teneur en HbF. L'analyse de la répartition des globules rouges en fonction de leur teneur en HbF permet d'identifier différents profils de réponse au traitement. A titre d'exemple les patients 11 et 24 (Tableaux 7 et 8), exprimant des %HbF identiques à J0 (6%) et à M6 (18 %) avec une TCMH à J0= 33,6 et 34,5 pg et à M6 38,9 et 42 pg (patients 11 et 24 respectivement), présentent 2 répartitions différentes. Chez le patient 11, on note un plus faible nombre de globules rouges avec une teneur en HbF de moins de 2pg avec une diminution d'environ 73% à M6 alors que le patient 24 présente un plus grand nombre de globules rouges avec une teneur en HbF de moins de 2pg avec une diminution d'environ 40% à M6. La distribution de l'HbF chez le patient 11 est décrite par une courbe bimodale à J0 et à M6 tandis que la distribution du patient 24 présente une distribution relativement homogène sur les faibles quantités d'HbF avec un net décalage de l'ensemble vers des quantités plus importantes.

**Patient 11**

| HbF (pg) | J0 (% GR) | J15-M1 (% GR) | M3-M4 (% GR) | ≥M6 (% GR) |
|---|---|---|---|---|
| [0 ; 2[ | 48,44 | 55,38 | 48,83 | 12,93 |
| [2 ; 4[ | 26,27 | 23,31 | 31,73 | 17,62 |
| [4 ; 6[ | 8,13 | 6,25 | 9,30 | 11,85 |
| [6 ; 8[ | 3,77 | 3,07 | 4,32 | 8,05 |
| [8 ; 10[ | 2,69 | 2,55 | 2,04 | 6,08 |
| [10; 15[ | 2,41 | 2,24 | 0,72 | 6,13 |
| [15 ; 20[ | 4,21 | 4,42 | 2,46 | 9,77 |
| ≥20 | 4,08 | 2,78 | 0,59 | 27,57 |

**Patient 24**

| HbF (pg) | J0 (% GR) | J15-M1 (% GR) | M3-M4 (% GR) | ≥M6 (% GR) |
|---|---|---|---|---|
| [0 ; 2[ | 60,82 | 53,17 | 44,56 | 36,98 |
| [2; 4[ | 9,38 | 11,57 | 24,93 | 25,28 |
| [4; 6 | 4,83 | 6,69 | 12,53 | 13,64 |

(suite)

| HbF (pg) | J0 (% GR) | J15-M1 (% GR) | M3-M4 (% GR) | ≥M6 (% GR) |
|---|---|---|---|---|
| [6 ; 8[ | 4,01 | 5,58 | 5,81 | 6,92 |
| [8 ; 10[ | 3,63 | 4,50 | 3,09 | 3,93 |
| [10 ; 15[ | 3,83 | 3,89 | 1,87 | 2,70 |
| [15 ; 20[ | 6,38 | 7,56 | 3,81 | 5,36 |
| ≥20 | 7,12 | 7,04 | 3,41 | 5,20 |

Tableaux 7 et 8: répartition de chaque globule rouge en fonction de leur teneur en classes d'HbF (par rapport aux globules rouges totaux) avant et au cours du traitement par hydroxyurée

Les données biologiques des patients 11 et 24 ont également été analysées (Tableau 9 ci-dessous.

Tableau 9 : Données biologiques des patients 11 et 24. HbF = Hémoglobine F ; RETIC = Réticulocytes ; LDH = Lactate déshydrogénase ; ASAT = Aspartate transaminase

| | Patient 11 (J0) | Patient 11 (M6) | Patient 24 (10) | Patient 24 (M6) | Différence relative en % J0 vs M6 (patient 11) | Différence relative en % J0 vs M6 (patient 24) |
|---|---|---|---|---|---|---|
| HbF (g/dl) | 6,2 | 7,1 | 7,6 | 8,7 | +14,5 | +14,5 |
| RETIC (g/L) | 275 | 63 | 271 | 116 | -77,1 | -57,2 |
| LDH (UI/L) | 905 | 383 | 509 | 303 | -57,7 | -40,5 |
| ASAT (UI/L) | 55 | 26 | 32 | 29 | -52,7 | -9,4 |

Les données biologiques ont montré que les patients 11 et 24 présentent des différences relatives identiques d'HbF (+14,5%) avant et après 6 mois de traitement par hydroxyurée. Ils présentent néanmoins des différentes importantes en termes de distribution d'HbF (Voir Tableau 8). Le patient 11, comparativement au patient 24, présente avant traitement (30) moins de GR ayant de fortes teneur en HbF protectrice (17% GR vs 25% GR > 6 pg d'HbF), ce qui pourrait expliquer un anémie plus importante (6,2 g/dl vs 7,6 g/dl) et une hémolyse plus importante (LDH à 905 UI/l vs 509 UI/l). En revanche l'augmentation des GR > 6 pg HbF chez le patient 11 a été plus marquée que chez le patient 24, à 6 mois de traitement (57% vs 24%), et pourrait expliquer une diminution de l'hémolyse plus importante sous traitement (RETIC: -77% vs -57%; LDH : - 57% vs -40,5%; ASAT -52% vs -9,4%).

Un autre exemple de répartition en fonction de la teneur en HbF de chaque globule rouge est représenté pour le patient 2. Le patient 2 présentait avant le traitement par Hydroxyurée une relative homogénéité de répartition de la teneur en HbF. Cependant à M6 la distribution s'est caractérisé par une nette augmentation des globules rouges contenant une teneur supérieure à 20pg au détriment de toutes les autres classes (Tableau 10). A noter que le %HbF varie de 6 à 25% entre 30 et M6, ce qui est similaire aux 2 exemples précédents.

Tableau 10: répartition des globules rouges en fonction de leur teneur en classes d'HbF (par rapport aux globules rouges totaux) avant et au cours du traitement par hydroxyurée pour le patient 2

| HbF (pg) | J0 (% GR) | J15-M1 (% GR) | M3-M4 (% GR) | ≥M6 (% GR) |
|---|---|---|---|---|
| [0 ;2[ | 22,43 | 9,21 | 5,29 | 4,98 |
| [2 ;4[ | 15,76 | 14,72 | 8,62 | 7,04 |
| [4 ;6[ | 12,85 | 9,52 | 10,61 | 5,53 |
| [6 ;8[ | 10,04 | 8,47 | 10,39 | 4,85 |
| [8 ;10[ | 7,84 | 7,35 | 8,75 | 4,56 |
| [10 ;15[ | 5,79 | 9,03 | 8,50 | 7,48 |

(suite)

| HbF (pg) | J0 (% GR) | J15-M1 (% GR) | M3-M4 (% GR) | ≥M6 (% GR) |
|---|---|---|---|---|
| [15 ;20[ | 11,05 | 14,27 | 14,54 | 9,75 |
| ≥20 | 14,24 | 27,43 | 33,30 | 55,81 |

6. Conclusion

Les données ont montré que les globules rouges présentant une teneur en HbF très basse diminuent fortement et que les globules rouges ayant une teneur en HbF supérieure à 20 pg augmentent de façon importante, jusqu'à 3 fois, après 6 mois de traitement par hydroxyurée.

**Revendications**

1.  Procédé de détermination *in vitro* de la teneur en hémoglobine F (HbF) de chaque globule rouge d'un ensemble de globules rouges comprenant les étapes de :

    a) isoler des globules rouges à partir d'un échantillon ;
    b) perméabiliser la membrane des globules rouges isolés ;
    c) marquer l'HbF des globules rouges obtenues à l'étape b) avec un anticorps anti-HbF conjugué à un fluorochrome ;
    d) mesurer par cytométrie de flux l'intensité de fluorescence (MFI) de chaque globule rouge d'un ensemble de globules rouges ;
    e) déterminer la teneur en hémoglobine F de chaque globule rouge de l'ensemble de globules rouges par la comparaison de l'intensité de fluorescence de chaque globule rouge avec une courbe standard permettant d'associer l'intensité de fluorescence mesurée pour un globule rouge à une teneur en HbF.

2.  Procédé selon la revendication 1, dans lequel la membrane des globules rouges isolés est fixée avant l'étape de perméabilisation.

3.  Procédé selon l'une quelconque des revendications 1 ou 2, dans lequel ledit échantillon est un échantillon sanguin, de préférence un échantillon sanguin humain.

4.  Procédé selon l'une quelconque des revendications 1 à 3, dans lequel ledit fluorochrome est la phycoérythrine (PE).

5.  Procédé selon l'une quelconque des revendications 1 à 4, dans lequel ledit anticorps anti-HbF est dirigé contre la chaîne gamma de l'HbF.

6.  Procédé selon l'une quelconque des revendications 1 à 5, dans lequel la membrane des globules rouges isolés est perméabilisée avec du dodécylsulfate de sodium.

7.  Procédé selon l'une quelconque des revendications 2 à 6, dans lequel la membrane des globules rouges isolés est fixée avec de l'azoture de sodium et/ou du formaldéhyde.

8.  Procédé selon l'une quelconque des revendications 1 à 7, dans lequel la teneur en hémoglobine F est déterminée pour chaque globule rouge d'un ensemble d'au moins 10 000 globules rouges, de préférence d'au moins 100 000 globules rouges, de préférence entre 50 000 et 100 000 globules rouges.

9.  Procédé de détermination *in vitro* de la quantité de globules rouges transfusés chez un patient, comprenant :

    a) la détermination de la teneur en hémoglobine F (HbF) de chaque globule rouge d'un ensemble de globules rouges d'un échantillon de globules rouges d'un patient selon le procédé de l'une quelconques des revendications 1 à 8, ;
    b) l'utilisation des résultats de l'étape a) pour déterminer la quantité de globules rouges transfusés chez un patient, lesdits globules rouges transfusés ayant une teneur en HbF substantiellement égale à zéro (= 0 pg).

**10.** Procédé *in vitro* de suivi de l'efficacité thérapeutique d'un traitement de la drépanocytose ou de la β-thalassémie, comprenant :

a) la détermination de la teneur en hémoglobine F (HbF) de chaque globule rouge d'un ensemble de globules rouges d'un échantillon de globules rouges d'un patient ayant subi un traitement de la drépanocytose ou de la β-thalassémie selon le procédé de l'une quelconque des revendications 1 à 8 ;
b) l'utilisation des résultats de l'étape a) dans la détermination de l'efficacité thérapeutique du traitement de la drépanocytose ou de la β-thalassémie, dans lequel une efficacité thérapeutique est observée lorsqu'au moins un pourcentage prédéterminé des globules rouges de l'ensemble de globules rouges a une teneur en HbF qui dépasse un seuil de référence de 2 pg ou plus et/ou lorsqu'on observe une diminution de la quantité de globules rouges ayant une teneur en HbF <2pg.

**11.** Procédé selon la revendication 10, dans lequel le seuil de référence de 2 pg ou plus est de 3 pg, 4 pg, 5 pg, 6 pg, 7 pg, ou 7,5 pg.

**Patentansprüche**

**1.** Verfahren zum *In-vitro*-Bestimmen des Gehalts an Hämoglobin F (HbF) jedes roten Blutkörperchens eines Satzes von roten Blutkörperchen, umfassend die folgenden Schritte:

a) Isolieren von roten Blutkörperchen aus einer Probe;
b) Permeabilisieren der Membran der isolierten roten Blutkörperchen,
c) Markieren des HbF der in Schritt b) erhaltenen roten Blutkörperchen mit einem Anti-HbF-Antikörper, der mit einem Fluorochrom konjugiert ist;
d) Messen der Fluoreszenzintensität (MFI) jedes roten Blutkörperchens eines Satzes von roten Blutkörperchen durch Durchflusszytometrie;
e) Bestimmen des Gehalts an Hämoglobin F jedes roten Blutkörperchens des Satzes von roten Blutkörperchen durch das Vergleichen der Fluoreszenzintensität jedes roten Blutkörperchens mit einer Standardkurve, die es ermöglicht, die für ein rotes Blutkörperchen gemessene Fluoreszenzintensität mit einem HbF-Gehalt in Verbindung zu bringen.

**2.** Verfahren nach Anspruch 1, wobei die Membran der isolierten roten Blutkörperchen vor dem Permeabilisierungsschritt fixiert wird.

**3.** Verfahren nach einem der Ansprüche 1 oder 2, wobei die Probe eine Blutprobe, vorzugsweise eine menschliche Blutprobe, ist.

**4.** Verfahren nach einem der Ansprüche 1 bis 3, wobei das Fluorochrom Phycoerythrin (PE) ist.

**5.** Verfahren nach einem der Ansprüche 1 bis 4, wobei der Anti-HbF-Antikörper gegen die Gamma-Kette von HbF gerichtet ist.

**6.** Verfahren nach einem der Ansprüche 1 bis 5, wobei die Membran der isolierten roten Blutkörperchen mit Natriumdodecylsulfat permeabilisiert wird.

**7.** Verfahren nach einem der Ansprüche 2 bis 6, wobei die Membran der isolierten roten Blutkörperchen mit Natriumazid und/oder Formaldehyd fixiert wird.

**8.** Verfahren nach einem der Ansprüche 1 bis 7, wobei der Gehalt an Hämoglobin F für jedes rote Blutkörperchen eines Satzes von mindestens 10.000 roten Blutkörperchen, vorzugsweise mindestens 100.000 roten Blutkörperchen, besonders bevorzugt zwischen 50.000 und 100.000 roten Blutkörperchen, bestimmt wird.

**9.** Verfahren zum *In-vitro*-Bestimmen der Menge an roten Blutkörperchen, die bei einem Patienten transfundiert wurden, umfassend:

a) Bestimmen des Gehalts an Hämoglobin F (HbF) jedes roten Blutkörperchens in einem Satz von roten Blutkörperchen in einer Probe roter Blutkörperchen eines Patienten gemäß dem Verfahren nach einem der Ansprüche 1 bis 8;
b) Verwenden der Ergebnisse von Schritt a), um die Menge an roten Blutkörperchen, die bei einem Patienten transfundiert wurden, zu bestimmen, wobei die transfundierten roten Blutkörperchen einen HbF-Gehalt von im Wesentlichen null (= 0 pg) aufweisen.

10. *In-vitro*-Verfahren zum Überwachen der therapeutischen Wirksamkeit einer Behandlung von Sichelzellenanämie oder β-Thalassämie, umfassend:

a) Bestimmen des Gehalts an Hämoglobin F (HbF) jedes roten Blutkörperchens in einem Satz von roten Blutkörperchen in einer Probe roter Blutkörperchen eines Patienten, der sich einer Behandlung von Sichelzellenanämie oder β-Thalassämie gemäß dem Verfahren eines der Ansprüche 1 bis 8 unterzogen hat;
b) Verwenden der Ergebnisse aus Schritt a) bei dem Bestimmen der therapeutischen Wirksamkeit der Behandlung von Sichelzellenanämie oder β-Thalassämie, wobei eine therapeutische Wirksamkeit beobachtet wird, wenn mindestens ein vorbestimmter Prozentsatz der roten Blutkörperchen des Satzes von roten Blutkörperchen einen HbF-Gehalt aufweist, der einen Referenzschwellenwert von 2 pg oder mehr überschreitet, und/oder wenn eine Verringerung der Menge an roten Blutkörperchen mit einem HbF-Gehalt von < 2 pg beobachtet wird.

11. Verfahren nach **Anspruch** 10, wobei der Referenzschwellenwert von 2 pg oder mehr 3 pg, 4 pg, 5 pg, 6 pg, 7 pg oder 7,5 pg beträgt.

## Claims

1. A method for determining *in vitro* the hemoglobin F (HbF) content of each red blood cell in a set of red blood cells comprising the steps of:

   a) isolating red blood cells from a sample;
   b) permeabilizing the membrane of isolated red blood cells;
   (c) labeling the HbF of the red blood cells obtained in step (b) with an anti-HbF antibody conjugated to a fluorochrome;
   (d) measuring, by flow cytometry, the fluorescence intensity (MFI) of each red blood cell in a set of red blood cells;
   (e) determining the haemoglobin F content of each red blood cell in the red blood cells set by comparing the fluorescence intensity of each red blood cell with a standard curve for associating the fluorescence intensity measured for a red blood cell with an HbF content.

2. The method of claim 1, wherein the membrane of the isolated red blood cells is fixed prior to the permeabilization step.

3. A method according to any one of claims 1 or 2, wherein said sample is a blood sample, preferably a human blood sample.

4. A method according to any one of claims 1 to 3, wherein said fluorochrome is phycoerythrin (PE).

5. A method according to any one of claims 1 to 4, wherein said anti-HbF antibody is directed against the gamma chain of HbF.

6. A method according to any one of claims 1 to 5, wherein the membrane of the isolated red blood cells is permeabilized with sodium dodecyl sulfate.

7. A method according to any one of claims 2 to 6, wherein the membrane of the isolated red blood cells is fixed with sodium azide and/or formaldehyde.

8. A method according to any one of claims 1 to 7, wherein the content of haemoglobin F is determined for each red blood cell in a set of at least 10,000 red blood cells, preferably of at least 100,000 red blood cells, preferably between 50,000 and 100,000 red blood cells.

9. A method for determining *in vitro* the amount of red blood cells transfused into a patient, comprising:

   (a) determining the haemoglobin F (HbF) content of each red blood cell in a set of red blood cells in a sample of red blood cells of a patient according to the method of any one of claims 1 to 8;
(b) using the results of step (a) to determine the quantity of red blood cells transfused into a patient, the said red blood cells transfused having an HbF content substantially equal to zero (= 0 pg).

10. In vitro *method* for monitoring the therapeutic efficacy of a treatment for sickle cell anemia or β-thalassemia, comprising:

(a) determining the haemoglobin F (HbF) content of each red blood cell of a set of red blood cells of a sample of red blood cells of a patient who has undergone treatment for sickle cell anemia or β-thalassemia according to the method of any one of claims 1 to 8;
(b) using the results of step (a) in determining the therapeutic efficacy of the treatment of sickle cell disease or β-thalassemia, in which therapeutic efficacy is observed when at least a predetermined percentage of the red blood cells in the red blood cell pool have an HbF content that exceeds a reference threshold of 2 pg or more and/or when a decrease in the amount of red blood cells with an HbF content <2pg is observed.

11. The method of claim 10, wherein the reference threshold of 2 pg or greater is 3 pg, 4 pg, 5 pg, 6 pg, 7 pg, or 7.5 pg.

# Figure 1

**Figure 2**

# Figure 3

A

B

Figure 4

Figure 4

Figure 4

**Figure 5**

## Figure 6 A

EP 3 535 589 B1

**Figure 6 B**

**Figure 6 C**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description

- *Bulletin Epidémiologique Hebdomadaire,* 03 Juillet 2012
- *Bulletin Epidémiologique Hebdomadaire,* 10 Mars 2015
- **BETKE K ; MARTI HR ; SCHLICHT I.** Estimation of small percentages of foetal haemoglobin. *Nature,* 1959, vol. 184 (24), 1877-8
- **NAVENOT JM ; MERGHOUB T ; DUCROCQ R ; MULLER JY ; KRISHNAMOORTHY R ; BLANCHARD D.** New method for quantitative détermination of fetal hemoglobin-containing red blood cells by flow cytometry: application to sickle-cell disease. *Cytometry,* 1998, vol. 32 (3), 186-90
- **MUNDEE Y et al.** Simplified flow cytométrie method for fetal hemoglobin containing red blood cells. *Cytometry,* 2000, vol. 42 (6), 389-393
- **STEINBERG et al.** *Blood,* 2014, vol. 123, 481-485
- Life Technologies SAS, Saint Aubin, France. DPBS. Gibco by life technologies Cat, vol. 14190-094